# EUROPEAN PATENT APPLICATION

(11) **EP 3 875 079 A1**
(43) Date of publication of application: **08.09.2021**
(21) Application number: 20160420.4
(22) Date of filing: 02.03.2020
(51) Int. Cl.: A61K 31/137, A61K 9/20, A61K 9/50, A61P 25/00, A61P 25/04

(54) **DOSAGE FORM PROVIDING PROLONGED RELEASE OF TAPENTADOL PHOSPHORIC ACID SALT**

(62) Divisional of application: 20183206.0
(71) Applicant: Grünenthal GmbH, 52078 Aachen (DE)
(72) Inventor: BERTRAM, Ulrike, 52066 Aachen (DE); REINHOLD, Ulrich, 52076 Aachen (DE); GROSSE, Christian, 52072 Aachen (DE); HARTMANN, Carmen, 50859 Köln (DE)
(74) Representative: Kutzenberger Wolff & Partner

(57) **Abstract**

The invention relates to a pharmaceutical dosage form providing prolonged release of Tapentadol, wherein Tapentadol is present in form of a salt with phosphoric acid (orthophosphoric acid), preferably as dihydrogenphosphate salt. The dosage form according to the invention provides improved prolonged release properties, is particularly resistant against ethanol induced dose dumping, and beyond such resistance provides additional safety features with regard to concomitant use of ethanol, e.g. alcoholic beverages.

## Description

The invention relates to a pharmaceutical dosage form providing prolonged release of Tapentadol, wherein Tapentadol is present in form of a salt with phosphoric acid (orthophosphoric acid), preferably as dihydrogenphosphate salt. The dosage form according to the invention provides improved prolonged release properties, is particularly resistant against ethanol induced dose dumping, and beyond such resistance provides additional safety features with regard to concomitant use of ethanol, e.g. alcoholic beverages.

Several extended release formulations can be subject to ethanol induced dose dumping (also referred to as alcohol induced dose dumping, alcoholic dose dumping, ethanolic dose dumping, and the like), which has been defined as the *"unintended, rapid drug release in a short period of time of the entire amount or a significant fraction of the drug contained in a modified-release dosage form".*

Of the numerous classes of drugs commercially available as extended release products, opioids, centrally acting drugs, and drugs with a narrow therapeutic index present high risks from dose dumping, despite being formulated in a manner that releases drug in a tailored or delayed fashion. Awareness of ethanol induced dose dumping has led to the withdrawal of a few marketed products by Regulatory Agencies, and black box warnings on others. Since then, significant effort has focused on proving the robustness of a formulation when co-ingested with alcohol. Patient risk is deemed to be low if the formulation and its performance is unimpaired by the presence of 0-40% alcohol under *in vivo* and *in vitro* conditions (see D'Souza et al., A review of in vivo and in vitro aspects of alcohol-induced dose dumping, AAPS Open (2017) 3:5, 1-20).

In the most pronounced case, co-ingestion of the previously available analgesic Palladone™ (Purdue Pharma; Hydromorphone hydrochloride extended-release capsules) with alcohol produced significantly higher plasma levels of hydromorphone (up to 16-fold greater), especially in the fasted state. This finding prompted its discontinuation and withdrawal from the market. *In vitro* studies of another long acting opioid, Avinza® (Pfizer Inc; Morphine sulfate extended release capsules), displayed accelerated release of morphine that was alcohol concentration dependent. Box warnings for Avinza®, as well as other extended-release/long-acting opioids, advise patients not to drink alcoholic beverages or use prescription or nonprescription medications containing alcohol during therapy, as it may result in the rapid release and absorption of a potentially fatal dose of opioid.

According to Jedinger et al., The design of controlled-release formulations resistant to alcohol-induced dose dumping - A review, Eur J Pharm Biopharm. 2014 Jul;87(2):217-26, the Food and Drug Administration currently recommends to assess the risk of alcohol-induced dose dumping for opioid and non-opioid drugs with a narrow therapeutic index. Especially, prolonged-release formulations, which offer a reduced dosing frequency and a prolonged therapeutic effect due to higher drug amounts, are of specific interest. For example, in the treatment of (chronic) pain controlled-release opioid dosage forms have been used as first choice formulations. However, if patients suffer from pain, they often turn to alcohol to cope with the pain-related stress and to reduce the pain perception.

The most deleterious effect is the first one which may arise due to rapid dose escalation. Thus, if the dosage form is consumed with ethanol, the drug release can increase immediately, resulting in an overdose and leading to respiratory depression followed by hypoxia and even death. Apart from side effects associated with ethanol, the mechanistic understanding of the oro-gastrointestinal absorption and hepatic metabolism is important. After oral administration of a dose dumping susceptible formulation co-ingested with ethanol, a major part of the drug is dissolved in the stomach immediately. After a sufficient gastric retention time, the entire amount of the dissolved drug is uncontrollably emptied into the small intestine. Thus, absorption occurs which may result in high plasma concentrations.

Thus, in order to avoid these effects, it is desirable to provide dosage forms with release characteristics such that release in aqueous ethanol is essentially not accelerated compared to release in non-ethanolic medium (resistance against ethanol induced dose dumping).

The above consequences of an initial burst of drug release, however, are not the only risk associated with co-ingestion of ethanol. It is known that the consumption of alcoholic beverages may prolong the gastric emptying rate and therefore the onset of drug absorption due to the caloric content of alcohol, which is comparable with the lightly-fed state after the consumption of a meal (for further details, see also Jedinger et al., The design of controlled-release formulations resistant to alcohol-induced dose dumping - A review, Eur J Pharm Biopharm. 2014 Jul;87(2):217-26).

The mechanisms by which alcohol alters the pharmacokinetic properties of long-acting opioids are poorly understood. Several studies have shown that concurrent use of alcohol increases the maximum plasma concentration (Cₘₐₓ) of certain opioids and decreases the time to Cₘₐₓ (tₘₐₓ), despite no evidence of dose dumping. Fatal poisonings involving prescription opioids are frequently associated with alcohol use and are likely due to combined CNS- and respiratory-depressant effects. It has been reported that opioids may significantly decrease the ventilatory response to hypercapnia when administered along with ethanol, even though no pharmacokinetic interaction was observed (see Gudin et al., Risks, Management, and Monitoring of Combination Opioid, Benzodiazepines, and/or Alcohol Use, Postgrad Mes 2013 125(4) 115-130).

Thus, even if pharmaceutical dosage forms can be provided that are resistant against ethanol induced dose dumping, i.e. that do not show accelerated dissolution in aqueous ethanol compared to non-ethanolic medium and therefore do not provide an initial burst of drug release when being co-ingested with ethanol, there are additional safety concerns that are related to the effects of ethanol. There is a demand for pharmaceutical dosage forms providing additional safety features that go beyond the mere resistance against ethanol induced dose dumping. In particular, it would be desirable to provide pharmaceutical dosage forms that do not just provide non-accelerated, but that provide decelerated dissolution in aqueous ethanol compared to non-ethanolic medium. It can be expected that such pharmaceutical dosage forms are less prone to risks associated with an interaction of the drug with co-ingested ethanol because at any given point in time, due to decelerated dissolution, less opioid is made available to the organism and might interact with the co-ingested ethanol.

Tapentadol (Nucynta®, Palexia®) is an oral opioid analgesic in the benzenoid class with a dual mechanism of action that is similar to tramadol; it is a µ-opioid receptor agonist and also inhibits the reuptake of norepinephrine. Tapentadol is currently available as oral dosage form containing Tapentadol hydrochloride salt and providing immediate release or prolonged release.

According to Farr et al., Effects of food and alcohol on the pharmacokinetics of an oral, extended-release formulation of hydrocodone in healthy volunteers, Clinical Pharmacology: Advances and Applications 2015:7 1-9, increased absorption following co-ingestion with alcohol has been observed with several extended-release opioid formulations. The authors provide the following comparison of alcohol interaction data for marketed extended-release opioid products in Table 3 on page 8:

| opioid | mean increase in Cₘₐₓ ratio relative to drug co-ingested with 0% alcohol | Maximum individual Cₘₐₓ ratio with 40% alcohol |
|---|---|---|
| Hydromorphone | 1.37 | 2.51 |
| Oxymorphone | 1.70 | 2.70 |
| Hydrocodone | 2.3 | 3.9 |
| Tapentadol 100 mg | 1.48 | 4.38 |
| Tapentadol 250 mg | 1.28 | 2.67 |
| Morphine/Naltrexone | 2.0 | 5.0 |
| Morphine | 1.0 | 4.54 |

According to FDA Draft Guidance on Tapentadol Hydrochloride (available at URL: *https:*//*www.accessdata.fda.gov*/*drugsatfda_docs*/*psg*/*Tapentadol_%20ERTabs*_*200533_RC09-10.pdf*), due to a concern of dose dumping of drug from this drug product when taken with alcohol, the Agency currently requests that additional dissolution testing be conducted using various concentrations of ethanol in the dissolution medium.

The pharmaceutical dosage forms of Tapentadol that are currently on the market already provide satisfactory resistance against ethanol induced dose dumping. An *in vivo,* cross-over study examined the effect of alcohol (240 mL of 40%) on the bioavailability of a single dose of 100 mg and 250 mg of Nucynta® extended-release tablets in 19 healthy, fasted volunteers. After co-administration of a 100 mg Nucynta® extended-release tablet and alcohol, the mean Cₘₐₓ value increased by 48% compared to control (consumed water instead of alcohol) with a range of 0.99-fold up to 4.38-fold. In addition, the 3 subjects (15%) with the highest Cₘₐₓ values were at least 2.3 times that of the control mean Cₘₐₓ value. The mean Tapentadol AUCₗₐₛₜ and AUC_{inf} were increased by 17%; the Tₘₐₓ and t_{1/2} were relatively unchanged. After co-administration of a 250 mg Nucynta® extended-release tablet and alcohol, the mean Cₘₐₓ value increased by 28% compared to control with a range of 0.90-fold up to 2.67-fold. The individual Cₘₐₓ value for 2 of these subjects (10%) were at least 2.6 times that of the control mean Cₘₐₓ value. The mean Tapentadol AUCₗₐₛₜ and AUC_{inf} were increased by 16%; the Tₘₐₓ and t_{1/2} were relatively unchanged.

Nonetheless, concomitant use of alcohol must be avoided since Nucynta® Extended-Release is expected to have additive effects when used in conjunction with alcohol (see PRODUCT MONOGRAPH, Nucynta® Extended-Release Tapentadol, October 28, 2013, Date of Revision: March 01,2018).

Therefore, in spite of providing satisfactory resistance against ethanol induced dose dumping, Nucynta® and Palexia® prescription information contain instructions not to consume alcohol or any products containing alcohol while taking the medication.

Several attempts have been reported in the literature with regard to the provision of pharmaceutical dosage forms containing strong opioids such as Tapentadol that have a further reduced potential for ethanol induced dose dumping.

WO 2015/004245 A1 relates to a tamper-resistant, oral pharmaceutical dosage form comprising a pharmacologically active ingredient having psychotropic action and an ethylene-vinyl acetate (EVA) polymer which provides resistance against solvent extraction, resistance against grinding, and resistance against dose-dumping in aqueous ethanol. The pharmacologically active ingredient is preferably selected from the group consisting of Oxycodone, Oxymorphone, Hydrocodone, Hydromorphone, Tramadol, Tapentadol, Morphine, Buprenorphine and the physiologically acceptable salts thereof. The dosage forms are preferably multiparticulate and preparation requires the application of heat and pressure. Preferably, the particles are extruded pellets, i.e. are produced by thermoforming with the assistance of an extruder. All examples relate to multiparticulate dosage forms where the particles are prepared by hot-melt extrusion.

WO 2018/219897 A1 relates to an oral pharmaceutical dosage form comprising a plurality of coated particles, wherein said coated particles comprise a core which comprises a Tapentadol component and which is coated with a controlled release coating material, wherein the controlled release coating material comprises a lubricant component and a polymer component, wherein the polymer component comprises one or more cellulose ethers and/or one or more acrylates, and wherein the pharmaceutical dosage form provides controlled release of the Tapentadol component. The controlled release coating material serves the purpose of controlling release of the Tapentadol component from the coated particles. Said controlled release coat of the controlled release coating material may form the outer surface of the particles or may be further overcoated with one or more layers of different or identical coating materials, e.g. in order to render the particles resistant against ethanol induced dose dumping. When the particles are prepared from nonpareils, they are coated with a drug coat comprising Tapentadol. These particles are additionally coated with a controlled release coat comprising controlled release coating material. When the coated particles provide resistance against ethanol induced dose dumping, they contain at least another two coating layers, namely an inner layer comprising an alginate salt, and an outer layer comprising an anionic acrylate polymer.

However, known concepts of avoiding ethanol induced dose dumping are not satisfactory in every respect and there is a demand for improved concepts. It would be desirable to provide oral dosage forms of Tapentadol that are easy to manufacture and do not require e.g. thermoforming or multiple coating steps. The oral dosage forms should preferably be provided in form of tablets, preferably monolithic tablets, i.e. should not require preparation of a multitude of particles. The preparation of the oral dosage forms should be possible on standard equipment on industrial high throughput scale, preferably by compression of powder mixtures possibly involving granulation (dry granulation or wet granulation), preferably, however, by direct compression of powder mixtures.

In particular, it would be desirable to provide additional safety features, namely to make pharmaceutical dosage forms available that do not merely show approximately the same dissolution profile in aqueous ethanol and in non-ethanolic medium, but which in view of the various effects of co-ingested ethanol on the pharmacological profile, provide an even more retarded dissolution in aqueous ethanol compared to non-ethanolic medium. Further, it would be desirable to reduce the amount of excipients that are necessary in order to provide retardant release of Tapentadol, e.g. with respect to oral administration twice daily.

It is an object of the invention to provide pharmaceutical dosage forms of Tapentadol that have advantages compared to the pharmaceutical dosage forms of the prior art. The pharmaceutical dosage forms should be safe and easy to manufacture in an economic manner, should provide advantageous patient compliance and resistance against ethanol induced dose dumping, should provide additional safety features with regard to co-ingestion of ethanol, particularly a more retarded dissolution in aqueous ethanol compared to non-ethanolic medium such that alterations of the pharmacological profile due to co-ingestion of ethanol are less likely to occur.

This object has been achieved by the subject-matter of the patent claims.

### SUMMARY OF THE INVENTION

The invention provides a pharmaceutical dosage form comprising Tapentadol for oral administration twice daily; wherein Tapentadol is present as a salt with phosphoric acid; wherein the dosage form provides prolonged release of Tapentadol; and wherein the weight equivalent dose of Tapentadol that is contained in the pharmaceutical dosage form is within the range of from 10 to 300 mg based on the free base of Tapentadol (Mᵣ = 221.3 g/mol).

It has been surprisingly found that salts of Tapentadol with phosphoric acid (H₃PO₄) are particularly useful for pharmaceutical dosage forms providing prolonged release of Tapentadol. It has been surprisingly found that dosage forms containing salts of Tapentadol with phosphoric acid *in vitro* provide slower release of Tapentadol in ethanolic medium than in non-ethanolic medium. These dosage forms are therefore expected to further prolong drug release *in vivo* when co-ingested with ethanol. This effect neither depends upon the particle size of the salts of Tapentadol with phosphoric acid, nor on the polymorphic form of the salts of Tapentadol with phosphoric acid. Further, this effect is observed from prolonged release matrices comprising different prolonged release matrix materials (e.g. hypromellose or Kollidon® SR), i.e. is attributable to the properties of the salt of Tapentadol with phosphoric acid as such.

Further, it has been surprisingly found that compared to Tapentadol hydrochloride, which is contained in the prolonged release pharmaceutical dosage forms of Tapentadol currently on the market, such salts of Tapentadol with phosphoric acid have a lower thermodynamic solubility as well as a slowed intrinsic dissolution rate. These dissolution properties of the phosphate salts of Tapentadol make them particularly suitable for the preparation of prolonged release dosage forms. Such dosage forms may contain reduced amounts of prolonged release matrix material in order to achieve the same dissolution profile as the currently marketed dosage forms containing Tapentadol hydrochloride.

Salts of Tapentadol with phosphoric acid are known, e.g. from WO 2010/096045, WO 2012/010316 A1, WO 2012/051246 A1 and WO 2017/182438 A1. However, none of these references addresses the use of the salts for avoidance of ethanol induced dose dumping.

Dosage forms providing prolonged release of Tapentadol are also known, e.g. from WO 03/035053 A1, WO 2006/002886 A1, and WO 2009/092601 A1. However, none of these references addresses avoidance of ethanol induced dose dumping and none of these references discloses salts of Tapentadol with phosphoric acid.

According to the instructions for use, the commercial Tapentadol tablets Palexia® retard contain Tapentadol as hydrochloride salt, whereas the tablet core additionally contains hypromellose, microcrystalline cellulose, highly disperse silicon dioxide, and magnesium stearate. Thus, Palexia® retard tablets contain a prolonged release matrix of hypromellose. The tablet cores are film coated with a composition comprising hypromellose, lactose monohydrate, talcum, macrogol 6000 and colorants. These tablets are in accordance with WO 03/035053 A1 and with the comparative examples contained herein in the experimental section.

### BRIEF DESCRIPTION OF THE DRAWINGS

Figure 1 shows the XRPD spectrum of Tapentadol dihydrogenphosphate (mixed form) as obtained according to Example 1.1.
Figure 2 shows the XRPD spectrum of Tapentadol dihydrogenphosphate hemihydrate as obtained according to Example 1.4.
Figure 3 shows the DSC plot of Tapentadol dihydrogenphosphate hemihydrate as obtained according to Example 1.4.
Figure 4 shows the XRPD spectrum of Tapentadol dihydrogenphosphate anhydrate as obtained according to Example 1.6.
Figure 5 shows the DSC plot of Tapentadol dihydrogenphosphate anhydrate as obtained according to Example 1.6.
Figure 6 shows three photographs of different particles size of the salt of Tapentadol with phosphoric acid (Figure 6A shows relatively fine particles, Figure 6B relatively coarse particles) and of the salt of Tapentadol with hydrochloric acid (Figure 6C).
Figure 7 compares the *in vitro* dissolution profiles of a conventional tablet containing Tapentadol hydrochloride and an inventive tablet containing salt of Tapentadol with phosphoric acid at in 0.1 N HCl (pH 1.0) and in 0.1 N HCl (pH 1.0) with 40 vol.-% ethanol.
Figure 8 compares the *in vitro* dissolution profiles of a conventional tablet containing Tapentadol hydrochloride and an inventive tablet containing salt of Tapentadol with phosphoric acid at pH 4.5 in aqueous buffer (without ethanol).
Figure 9 compares the *in vitro* dissolution profiles of a conventional tablet containing Tapentadol hydrochloride at pH 1.0, pH 4.5, pH 6.8 in each case in aqueous buffer without ethanol and at pH 1.0 in 40 vol.-% ethanol.
Figure 10 compares the *in vitro* dissolution profiles of an inventive tablet containing salt of Tapentadol with phosphoric acid at pH 1.0, pH 4.5, pH 6.8 in each case without ethanol and at pH 1.0 in 40 vol.-% ethanol.
Figure 11 compares the *in vitro* dissolution profiles of an inventive tablet containing salt of Tapentadol with phosphoric acid in 0.1N HCL (pH 1.0) without ethanol and in 0.1N HCL (pH 1.0) with 40 vol.-% ethanol.

### DETAILED DESCRIPTION OF THE INVENTION

A first aspect of the invention relates to a pharmaceutical dosage form comprising Tapentadol for oral administration twice daily; wherein Tapentadol is present as a salt with phosphoric acid; wherein the dosage form provides prolonged release of Tapentadol; and wherein the weight equivalent dose of Tapentadol that is contained in the pharmaceutical dosage form is within the range of from 10 to 300 mg based on the free base of Tapentadol.

Tapentadol, i.e. (-)-(1R,2R)-3-(3-dimethylamino-1-ethyl-2-methyl-propyl)-phenol, is a synthetic, centrally acting analgesic which is effective in the treatment of moderate to severe, acute or chronic pain. The synthesis of the free base of Tapentadol is known e.g. from EP-A 693 475.

The pharmaceutical dosage form according to the invention contains Tapentadol as a salt with phosphoric acid. While it is contemplated that the pharmaceutical dosage form according to the invention may contain mixtures of different salts of Tapentadol or mixtures of salt(s) with the free base of Tapentadol (e.g. the non-salt form of Tapentadol), preferably the total amount of Tapentadol that is contained in the pharmaceutical dosage form is present as a salt with phosphoric acid.

Salts of Tapentadol with phosphoric acid according to the invention principally include the orthophosphate salts (PO₄³⁻), the monohydrogenphosphate salts (HPO₄²⁻), and the dihydrogenphosphate (H₂PO₄⁻). Condensation products of phosphoric acid such as metaphosphate salts or pyrophosphate salts are also contemplated, but less preferred. The Tapentadol dihydrogenphosphate salts are particularly preferred.

Preferably, the salt of Tapentadol with phosphoric acid is the dihydrogenphosphate salt of Tapentadol, which may optionally be solvated (e.g. hydrated) or ansolvated (e.g. anhydrated). Preferably, the dihydrogenphosphate salt of Tapentadol is selected from the group consisting of ansolvated dihydrogenphosphate salt of Tapentadol, anhydrated dihydrogenphosphate salt of Tapentadol, solvated dihydrogenphosphate salt of Tapentadol, hydrated dihydrogenphosphate salt of Tapentadol, and any mixtures of the foregoing. The salt of Tapentadol may be present in form of a single polymorph or as a mixture of different polymorphs in any mixing ratio.

In a preferred embodiment, the salt of Tapentadol with phosphoric acid is the dihydrogenphosphate salt which is present in essentially pure crystalline form of Tapentadol dihydrogenphosphate hemihydrate salt.

In a preferred embodiment, the salt of Tapentadol with phosphoric acid is the dihydrogenphosphate salt which is present in essentially pure crystalline form of Tapentadol dihydrogenphosphate anhydrate salt.

In a preferred embodiment, the salt of Tapentadol with phosphoric acid is a crystalline dihydrogenphosphate salt having characteristic X-ray powder diffraction peaks at 5.1 , 14.4, 17.7, 18.3 and 21.0 degrees 2Θ (± 0.2 degrees 2Θ), preferably being characterized by one or more further XRPD diffraction peak(s) at 8.7, 17.6, 20.3, 22.1 and/or 23.4 degrees 2Θ (± 0.2 degrees 2 Θ). Alternatively, the crystalline dihydrogenphosphate salt can be characterized by the XRPD diffraction peak(s) at degrees 2Θ ± 0.2 degrees 2Θ (intensity %): 5.1 (100), 8.7 (10), 10.1 (1), 12.5 (3), 13.4 (4), 14.4 (37), 15.3 (4), 17.6 (12), 17.7 (15), 18.3 (27), 19.1 (7), 20.3 (11), 21.0 (25), 21.6 (9), 22.1 (18), 23.4 (14), 24.8 (7), 25.0 (17), 25.3 (12), 26.0 (9), 26.5 (7), 26.8 (9), 28.0 (5), 28.4 (4), 28.9 (10), 29.2 (6), 29.4 (4), and 29.6 (2). Alternatively, the crystalline dihydrogenphosphate salt can be characterized by the XRPD diffraction peak(s) at degrees 2Θ ± 0.2 degrees 2Θ: 5.1 , 8.7, 10.1 , 12.5, 13.4, 14.4, 15.3, 17.6, 17.7, 18.3, 19.1 , 20.3, 21.0, 21.6, 22.1 , 23.4, 24.8, 25.0 and 25.3.

In another preferred embodiment, the salt of Tapentadol with phosphoric acid is a crystalline dihydrogenphosphate salt having characteristic X-ray powder diffraction peaks at 5.1 , 14.3, 17.6, 18.5 and 21.1 degrees 2Θ (± 0.2 degrees 2Θ); preferably being characterized by one or more further XRPD diffraction peak(s) at 8.9, 20.5, 22.4, 23.5 and/or 24.3 degrees 2Θ (± 0.2 degrees 2Θ). Alternatively, the crystalline dihydrogenphosphate salt can be characterized by the XRPD diffraction peak(s) at degrees 2Θ ± 0.2 degrees 2Θ (intensity %): 5.1 (100), 8.9 (3), 12.4 (5), 13.5 (4), 14.3 (42), 15.2 (4), 17.6 (21), 18.5 (18), 19.1 (7), 20.5 (14), 21.1 (28), 21.7 (9), 22.4 (14), 23.5 (15), 24.3 (6), 24.9 (19), 25.1 (16), 25.8 (3), 26.2 (12), 26.4 (13), 26.7 (5), 27.3 (2), 28.2 (5), 28.8 (8), 29.1 (11), 29.4 (5), and 29.6 (5). Alternatively, the crystalline dihydrogenphosphate salt can be characterized by the XRPD diffraction peak(s) at degrees 2Θ ± 0.2 degrees 2Θ: 5.1 , 8.9, 12.4, 13.5, 14.3, 15.2, 17.6, 18.5, 19.1 , 20.5, 21.1 , 21.7, 22.4, 23.5, 24.3, 24.9, and 25.1.

Preferably, the salt of Tapentadol with phosphoric acid has an average particle size, expressed as surface mean D[3,2] (Sauter Mean Diameter) and determined by laser diffraction in the dry mode in accordance with ISO 13320:2020, within the range of 5.0 to 500 µm, preferably 50±40 µm, or 75±40 µm, or 100±40 µm, or 125±40 µm, or 150±40 µm, or 175±40 µm, or 200±40 µm, or 225±40 µm, or 250±40 µm, or 275±40 µm, or 300±40 µm, or 325±40 µm, or 350±40 µm, or 375±40 µm, or 400±40 µm, or 425±40 µm, or 450±40 µm.

In a preferred embodiment, the salt of Tapentadol with phosphoric acid is present in crystalline form. In another preferred embodiment, the salt of Tapentadol with phosphoric acid is present in amorphous form. It is further contemplated that the pharmaceutical dosage form may contain mixtures of crystalline salt of Tapentadol with phosphoric acid with amorphous salt of Tapentadol with phosphoric acid in any mixing ratio. Preferably, essentially the total amount of the salt of Tapentadol with phosphoric acid that is present in the pharmaceutical dosage form is crystalline.

For the purpose of the specification, the dihydrogenphosphate salt of Tapentadol is to be regarded as the acid addition salt of 1 mole orthophosphoric acid to 1 mole of Tapentadol. It is believed that one proton of orthophosphoric acid (H₃PO₄) protonates the amino group of Tapentadol thereby forming an ammonium ion, whereas the remainder of orthophosphoric acid, i.e. the dihydrogenphosphate anion (H₂PO₄⁻), forms the counterion:

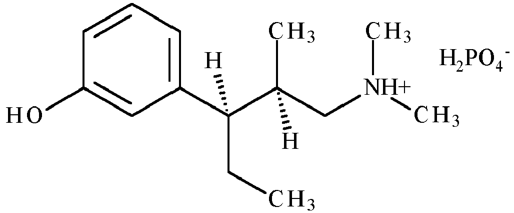

For the purpose of the specification, reference to "Tapentadol" shall include the salt of Tapentadol with phosphoric acid.

The salt of Tapentadol with phosphoric acid, preferably the dihydrogenphosphate salt of Tapentadol, may be present in form of any solvate, e.g. hydrate, ansolvate, e.g. anhydrate, and polymorphic form, e.g. any crystalline form and/or amorphous form. With regard to these forms and the preparation thereof reference is made to WO 2012/010316 A1 and WO 2017/182438 A1.

The weight equivalent dose of Tapentadol that is contained in the pharmaceutical dosage form according to the invention is within the range of from 10 to 300 mg based on the free base of Tapentadol, e.g. 25 mg, 50 mg, 100 mg, 150 mg, 200 mg, or 250 mg. The free base of Tapentadol (i.e. the free molecule) has a molecular weight of 221.3 g/mol. Thus, 10 mg of the free base of Tapentadol correspond to 0.0452 mmol, whereas 300 mg of the free base of Tapentadol correspond to 1.3556 mmol. Thus, in other words, the pharmaceutical dosage form contains a mole equivalent dose of Tapentadol within the range of from 0.0452 to 1.3556 mmol.

The hydrochloride salt of Tapentadol (anhydrate) has a molecular weight of 257.8 g/mol, whereas the dihydrogenphosphate salt of Tapentadol (anhydrate) has a molecular weight of 319.3 g/mol. Thus, when the pharmaceutical dosage form according to the invention contains e.g. a weight equivalent dose of 100 mg based on the free base of Tapentadol (0.4518 mmol), it actually contains 144.3 mg dihydrogenphosphate salt of Tapentadol (0.4518 mmol).

Unless expressly stated otherwise, dose information for Tapentadol is thus expressed as equivalent weight relative to the free base of Tapentadol, i.e. the non-salt form, the non-solvate form, the non-cocrystal form, and the non-aggregate form of Tapentadol with any other molecules. It is contemplated that the form of Tapentadol that is actually contained in the pharmaceutical dosage form, i.e. the salt of Tapentadol with phosphoric acid, may be present in form of any polymorph and/or solvate and/or cocrystal and/or any other aggregate of such salt with other molecules.

Unless expressly stated otherwise, all percentages are weight percent and are related to the total weight of the pharmaceutical dosage form. When the pharmaceutical dosage form is coated, the weight of the coating is included in the total weight of the pharmaceutical dosage form.

While it is contemplated that besides Tapentadol the pharmaceutical dosage form according to the invention may contain additional pharmacologically active ingredients, Tapentadol is preferably the sole pharmacologically active ingredient that is contained in the pharmaceutical dosage form.

Preferably, Tapentadol is homogeneously distributed over the pharmaceutical dosage form according to the invention.

The pharmaceutical dosage form according to the invention is devoted for oral administration, preferably by swallowing the pharmaceutical dosage form as a whole. Thus, the pharmaceutical dosage form according to the invention is preferably not devoted for buccal or sublingual administration where the pharmaceutical dosage form would be devoted to remain within the oral cavity.

The pharmaceutical dosage form according to the invention is devoted for administration twice daily. Thus, the pharmaceutical dosage form according to the invention contains 50% of the daily dose of Tapentadol that is intended for administration in order to bring about the desired therapeutic effect.

Administration twice daily may proceed at intervals of about every 12 hours, although such regimen is not to be strictly followed. For the purpose of the specification, twice daily shall also encompass any administration regimen where two pharmaceutical dosage forms according to the invention are administered during a period of about 24 hours, where the two administrations must be separated from one another by at least 4 hours, preferably at least 8 hours.

The pharmaceutical dosage form according to the invention provides prolonged release of Tapentadol. For the purpose of the specification, prolonged release means not immediate release. Prolonged release includes controlled release, delayed release, extended release, staggered release, repeat action release, sustained release and evenly sustained release. Prolonged release preferably means a release with a reduced release rate, to obtain a therapeutic effect upright, to reduce toxic effects or for some other therapeutic purpose.

Prolonged release may be based upon different technologies that are known to the skilled person. In a preferred embodiment, prolonged release is based upon prolonged release coating materials with which the pharmaceutical dosage form as such or with which a multitude of particles may be coated. In another preferred embodiment, prolonged release is based upon a prolonged release matrix in which Tapentadol is preferably embedded. It is further contemplated in accordance with the invention that prolonged release may be achieved by alternative concepts such as ion exchange resins, osmotic dosage forms, and the like.

Preferably, the pharmaceutical dosage form according to the invention after oral administration provides plasma levels of Tapentadol providing pain relief (analgesia) for a duration of at least 6 hours, preferably at least 8 hours, more preferably at least 10 hours, most preferably at least 12 hours.

Preferably, the pharmaceutical dosage form according to the invention provides an *in vitro* dissolution profile, when measured by the USP Paddle Method at 50 rpm in 900 ml aqueous phosphate buffer at pH 6.8 at 37° C, wherein
- after 0.5 hour 20±20 wt.-%; preferably 20±15 wt.-%; more preferably 20±10 wt.-%;
- after 4 hours 60±20 wt.-%; preferably 60±15 wt.-%; more preferably 60±10 wt.-%; and
- after 12 hours at least 60 wt.-%; preferably at least 70 wt.-%; more preferably at least 80 wt.-%
of the Tapentadol that was originally contained in the dosage form have been released.

Preferably, the pharmaceutical dosage form according to the invention provides an *in vitro* dissolution profile, when measured by the USP Paddle Method at 50 rpm in 900 ml aqueous phosphate buffer at pH 6.8 at 37° C, wherein
- after 1 hour 25±15 wt.-%; preferably 25±10 wt.-%; more preferably 25±5.0 wt.-%;
- after 2 hours 35±20 wt.-%; preferably 35±10 wt.-%; more preferably 35±5.0 wt.-%;
- after 4 hours 50±20 wt.-%; preferably 50±10 wt.-%; more preferably 50±5.0 wt.-%;
- after 8 hours 80±20 wt.-%; preferably 80±10 wt.-%; more preferably 80±5.0 wt.-%;
of the Tapentadol that was originally contained in the dosage form have been released.

Preferably, the pharmaceutical dosage form according to the invention provides an *in vitro* dissolution profile, when measured by the USP Paddle Method at 50 rpm in 900 ml aqueous buffer at pH 4.5 at 37° C, wherein
- after 0.5 hour 20±20 wt.-%; preferably 20±15 wt.-%; more preferably 20±10 wt.-%;
- after 4 hours 60±20 wt.-%; preferably 60±15 wt.-%; more preferably 60±10 wt.-%; and
- after 12 hours at least 60 wt.-%; preferably at least 70 wt.-%; more preferably at least 80 wt.-%
of the Tapentadol that was originally contained in the dosage form have been released.

Preferably, the pharmaceutical dosage form according to the invention provides an *in vitro* dissolution profile, when measured by the USP Paddle Method at 50 rpm in 900 ml aqueous buffer at pH 4.5 at 37° C, wherein
- after 1 hour 25±15 wt.-%; preferably 25±10 wt.-%; more preferably 25±5.0 wt.-%;
- after 2 hours 35±20 wt.-%; preferably 35±10 wt.-%; more preferably 35±5.0 wt.-%;
- after 4 hours 50±20 wt.-%; preferably 50±10 wt.-%; more preferably 50±5.0 wt.-%;
- after 8 hours 80±20 wt.-%; preferably 80±10 wt.-%; more preferably 80±5.0 wt.-%;
of the Tapentadol that was originally contained in the dosage form have been released.

Preferably, the pharmaceutical dosage form according to the invention provides an *in vitro* dissolution profile, when measured by the USP Paddle Method at 50 rpm in 900 ml 0.1 N HCl at pH 1.0 and 37 °C, wherein
- after 0.5 hour 20±20 wt.-%; preferably 20±15 wt.-%; more preferably 20±10 wt.-%;
- after 4 hours 60±20 wt.-%; preferably 60±15 wt.-%; more preferably 60±10 wt.-%; and
- after 12 hours at least 60 wt.-%; preferably at least 70 wt.-%; more preferably at least 80 wt.-%
of the Tapentadol that was originally contained in the dosage form have been released.

Preferably, the pharmaceutical dosage form according to the invention provides an *in vitro* dissolution profile, when measured by the USP Paddle Method at 50 rpm in 900 ml 0.1 N HCl at pH 1.0 and 37 °C, wherein
- after 1 hour 25±10 wt.-%; preferably 25±10 wt.-%; more preferably 25±5.0 wt.-%;
- after 2 hours 40±30 wt.-%; preferably 40±10 wt.-%; more preferably 40±5.0 wt.-%;
- after 4 hours 60±20 wt.-%; preferably 60±10 wt.-%; more preferably 60±5.0 wt.-%;
- after 8 hours 80±20 wt.-%; preferably 80±10 wt.-%; more preferably 80±5.0 wt.-%;
of the Tapentadol that was originally contained in the dosage form have been released.

Preferably, the pharmaceutical dosage form according to the invention provides resistance against ethanol induced dose dumping.

Preferably, the pharmaceutical dosage form according to the invention provides slower *in vitro* dissolution of Tapentadol in aqueous medium containing ethanol than in aqueous medium not containing ethanol.

Preferably, the pharmaceutical dosage form according to the invention provides slower dissolution of Tapentadol in 0.1 N HCl containing 5.0 vol.-% of ethanol (pH 1.0) than in 0.1 N HCl not containing 5.0 vol.-% of ethanol (pH 1.0), in each case when measured by the USP Paddle Method at 50 rpm in 900 ml at 37° C.

Preferably, the pharmaceutical dosage form according to the invention provides slower dissolution of Tapentadol in 0.1 N HCl containing 20 vol.-% of ethanol (pH 1.0) than in 0.1 N HCl not containing 20 vol.-% of ethanol (pH 1.0), in each case when measured by the USP Paddle Method at 50 rpm in 900 ml at 37° C.

Preferably, the pharmaceutical dosage form according to the invention provides slower dissolution of Tapentadol in 0.1 N HCl containing 40 vol.-% of ethanol (pH 1.0) than in 0.1 N HCL not containing 40 vol.-% of ethanol (pH 1.0), in each case when measured by the USP Paddle Method at 50 rpm in 900 ml at 37° C.

In preferred embodiments of the pharmaceutical dosage form according to the invention, the weight equivalent dose of Tapentadol that is contained in the pharmaceutical dosage form amounts to 25 mg, 50 mg, or 100 mg, in each case based on the free base of Tapentadol. Preferably, the pharmaceutical dosage form according to the invention has a total weight within the range of from 150 to 750 mg.

In preferred embodiments of the pharmaceutical dosage form according to the invention, the weight equivalent dose of Tapentadol that is contained in the pharmaceutical dosage form amounts to 150 mg, 200 mg or 250 mg, in each case based on the free base of Tapentadol. Preferably, the pharmaceutical dosage form according to the invention has a total weight within the range of from 300 to 1200 mg.

Preferably, the pharmaceutical dosage form according to the invention comprises one, two or more physiologically acceptable excipients.

Pharmaceutical excipients are known to the skilled person (cf. e.g. R. C. Rowe et al., Handbook of Pharmaceutical Excipients, Pharmaceutical Press; 6th edition 2009; E.-M. Hoepfner et al., Fiedler-Encyclopedia of Excipients, Editio Cantor, 6th edition 2008). For the purpose of the specification, a "pharmaceutical excipient" is preferably to be regarded as any pharmacologically inactive substance typically used as a carrier for the active ingredients of a medication. The pharmaceutical excipient may have a physiological effect, e.g. like a vitamin, but not a pharmacological effect, like a drug. Typical examples of pharmaceutical excipients include antiadherents, binders, coating materials, disintegrants, fillers, diluents, flavors, colorants, glidants, lubricants, preservatives, sorbents, surfactants, sweeteners, dyes, pigments, and the like.

Any of the foregoing excipients can be divided into sub-groups. For example, preservatives can be divided into antioxidants, buffers, antimicrobial substances and the like; whereas binders can be divided into solution binders and dry binders. Several excipients simultaneously exhibit different properties so that they can serve different purposes. For example, polyethylene glycol can be used as binder, plasticizer and the like.

Preferably, the pharmaceutical dosage form according to the invention, preferably the prolonged release matrix, comprises a binder.

Preferably, the binder is selected from the group consisting of cellulose, magnesium-aluminum silicates (e.g. bentonite), mono-, oligo- and poylsaccharides (e.g. dextrose, lactose, mannose), sugar alcohols (e.g. lactitol, mannitol), starches (e.g. pregelatinized starch, hydrolyzed starch, modified starch), calcium phosphate, polyvinylpyrrolidone, and vinylpyrrolidone/vinyl acetate copolymers; preferably microcrystalline cellulose; more preferably silicified microcrystalline cellulose.

Preferably, the weight content of the binder is at least 5.0 wt.-%, more preferably at least 10 wt.-%, still more preferably at least 15 wt.-%, yet more preferably at least 20 wt.-%, even more preferably at least 25 wt.-%, most preferably at least 30 wt.-%, and in particular at least 35 wt.-%, in each case relative to the total weight of the pharmaceutical dosage form.

Preferably, the weight content of the binder is at most 85 wt.-%, more preferably at most 82.5 wt.-%, still more preferably at most 80 wt.-%, yet more preferably at most 77.5 wt.-%, even more preferably at most 75 wt.-%, most preferably at most 72.5 wt.-%, and in particular at most 70 wt.-%, in each case relative to the total weight of the pharmaceutical dosage form.

Preferably, the weight content of the binder is within the range of from 52±30 wt.-%, more preferably 52±27.5 wt.-%, still more preferably 52±25 wt.-%, yet more preferably 52±22.5 wt.-%, even more preferably 52±20 wt.-%, most preferably 52±17.5 wt.-%, and in particular 52±15 wt.-%, in each case relative to the total weight of the pharmaceutical dosage form.

When the pharmaceutical dosage form contains more than one binder, the above percentages refer to the total content of all binders that are contained in the pharmaceutical dosage form.

Preferably, the pharmaceutical dosage form according to the invention comprises a lubricant.

Preferably, the lubricant is selected from the group consisting of salts of fatty acids (e.g. magnesium stearate, calcium stearate, zinc stearate), fatty acids (e.g. stearic acid, palmitic acid), glyceryl fatty acid esters (e.g. glyceryl monostearate, glyceryl monobehenate, glyceryl dibehenate, glyceryl tribehenate), sorbitan monostearate, sucrose monopalmitate, sodium stearyl fumarate, hydrated magnesium silicate, and talc; preferably magnesium stearate.

Preferably, the weight content of the lubricant is at least 0.20 wt.-%, more preferably at least 0.25 wt.-%, still more preferably at least 0.30 wt.-%, yet more preferably at least 0.35 wt.-%, even more preferably at least 0.40 wt.-%, most preferably at least 0.45 wt.-%, and in particular at least 0.50 wt.-%, in each case relative to the total weight of the pharmaceutical dosage form.

Preferably, the weight content of the lubricant is at most 3.0 wt.-%, more preferably at most 2.8 wt.-%, still more preferably at most 2.6 wt.-%, yet more preferably at most 2.40 wt.-%, even more preferably at most 2.20 wt.-%, most preferably at most 2.00 wt.-%, and in particular at most 1.80 wt.-%, in each case relative to the total weight of the pharmaceutical dosage form.

Preferably, the weight content of the lubricant is within the range of from 0.1 to 1.0 wt.-%, more preferably 0.50±0.45 wt.-%, still more preferably 0.50±0.40 wt.-%, yet more preferably 0.50±0.35 wt.-%, even more preferably 0.50±0.30 wt.-%, most preferably 0.50±0.25 wt.-%, and in particular 0.50±0.20 wt.-%, in each case relative to the total weight of the pharmaceutical dosage form.

In preferred embodiments of the pharmaceutical dosage form according to the invention, the dosage form contains a prolonged release coating which comprises a prolonged release coating material selected from the group consisting of hydrophobic cellulose ethers, acrylic polymers, shellac, zein, hydrophobic waxy-type products, and mixtures thereof.

It is contemplated that the dosage form is a monolith comprising such a prolonged release coating. In a preferred embodiment, the dosage form is multiparticulate wherein the individual particles (granules, pellets and the like) comprising such a prolonged release coating.

The number of particles that are contained in the dosage form is not particularly limited any may range from 1, 2, 3, 4 or 5 to 10, 20, 30, 40 to 100, 200, and more.

Preferably, the particles are essentially of the same weight, size and composition.

In a preferred embodiment, the particles contain a core comprising essentially the total amount of Tapentadol, optionally together with one or more excipients, and a prolonged release coating encapsulating said core.

In another preferred embodiment, the particles contain an inert core not containing any Tapentadol (e.g. nonpareils), a drug coating layer encapsulating said core and comprising essentially the total amount of Tapentadol, optionally together with one or more excipients, and a prolonged release coating encapsulating said core and said drug coating layer.

Preferably, the prolonged release coating material is selected from the group consisting of ethylcellulose, acrylic acid and methacrylic acid copolymers, methacrylic acid copolymers, methyl methacrylate copolymers, ethoxyethyl methacrylates, cynaoethyl methacrylate, methyl methacrylate, copolymers, methacrylic acid copolymers, methyl methacrylate copolymers, methyl methacrylate copolymers, methyl methacrylate copolymers, methacrylic acid copolymer, aminoalkyl methacrylate copolymer, methacrylic acid copolymers, methyl methacrylate copolymers, poly(acrylic acid), poly(methacrylic acid, methacrylic acid alkylamide copolymer, poly(methyl methacrylate), poly(methacrylic acid) (anhydride), methyl methacrylate, polymethacrylate, methyl methacrylate copolymer, poly(methyl methacrylate), poly(methyl methacrylate) copolymer, polyacrylamide, aminoalkyl methacrylate copolymer, poly(methacrylic acid anhydride), and glycidyl methacrylate copolymers.

In preferred embodiments, the acrylic polymer is comprised of one or more ammonio methacrylate copolymers, i.e. copolymers of acrylic and methacrylic acid esters with a low content of quaternary ammonium groups. In order to obtain a desirable dissolution profile, it may be necessary to incorporate two or more ammonio methacrylate copolymers having differing physical properties, such as different molar ratios of the quaternary ammonium groups to the neutral (meth)acrylic esters.

Preferably, the coating is made from an aqueous dispersion or from an organic dispersion or from an organic solution of a hydrophobic polymer. Preferably, the coating comprises an effective amount of a plasticizer that is also present in the aqueous dispersion of hydrophobic polymer. The plasticizer further improves the physical properties of the film. For example, because ethylcellulose has a relatively high glass transition temperature and does not form flexible films under normal coating conditions, it is necessary to plasticize the ethylcellulose before using the same as a coating material. Generally, the amount of plasticizer included in a coating solution is based on the concentration of the film-former, e.g., most often from about 1 to about 50 percent by weight of the film-former.

Examples of suitable plasticizers for ethylcellulose include water insoluble plasticizers such as dibutyl sebacate, diethyl phthalate, triethyl citrate, tributyl citrate, and triacetin, although it is possible that other water-insoluble plasticizers (such as acetylated monoglycerides, phthalate esters, castor oil, etc.) may be used. Triethyl citrate is an especially preferred plasticizer for the aqueous dispersions of ethyl cellulose of the present invention.

Examples of suitable plasticizers for the acrylic polymers of the present invention include, but are not limited to citric acid esters such as triethyl citrate NF XVI, tributyl citrate, dibutyl phthalate, and possibly 1,2-propylene glycol. Other plasticizers which have proved to be suitable for enhancing the elasticity of the films formed from acrylic films such as Eudragit® RL/RS lacquer solutions include polyethylene glycols, propylene glycol, diethyl phthalate, castor oil, and triacetin. Triethyl citrate is an especially preferred plasticizer for the aqueous dispersions of ethyl cellulose of the present invention.

The addition of talc reduces the tendency of the aqueous dispersion to stick during processing, and acts as a polishing agent.

In addition to modifying the dissolution profile by altering the relative amounts of different acrylic resin lacquers, the dissolution profile of the ultimate product may also be modified, for example, by increasing or decreasing the thickness of the retardant coating. When the aqueous dispersion of hydrophobic polymer is used to coat inert pharmaceutical beads, a plurality of the resultant stabilized solid prolonged-release beads may thereafter be placed in a gelatin capsule in an amount sufficient to provide an effective prolonged-release dose when ingested and contacted by gastric fluid.

The prolonged-release profile can be altered, for example, by varying the amount of overcoating with the aqueous dispersion of hydrophobic polymer, altering the manner in which the plasticizer is added to the aqueous dispersion of hydrophobic polymer, by varying the amount of plasticizer relative to hydrophobic polymer, by the inclusion of additional ingredients or excipients, by altering the method of manufacture, etc.

The coating preferably contains, in addition to the film-former, plasticizer, and solvent system (i.e., water), a colorant to provide elegance and product distinction. Suitable ingredients for providing color to the formulation when an aqueous dispersion of an acrylic polymer is used include titanium dioxide and color pigments, such as iron oxide pigments. The incorporation of pigments, may, however, increase the retard effect of the coating.

The plasticized aqueous dispersion or organic dispersion or organic solution of hydrophobic polymer may be applied onto the substrate comprising Tapentadol by spraying using any suitable spray equipment known in the art. In a preferred method, a Wurster fluidized-bed system is used in which an air jet, injected from underneath, fluidizes the core material and effects drying while the acrylic polymer coating is sprayed on. A sufficient amount of the aqueous dispersion of hydrophobic polymer to obtain a predetermined controlled-release of Tapentadol when said coated substrate is exposed to aqueous solutions, e.g. gastric fluid, is preferably applied, taking into account the manner of incorporation of the plasticizer, etc. After coating with the hydrophobic polymer, a further overcoat of a film-former, such as Opadry®, is optionally applied to the beads. This overcoat is provided, if at all, in order to substantially reduce agglomeration of the beads.

The release of Tapentadol from the prolonged-release formulation can be further influenced, i.e., adjusted to a desired rate, by the addition of one or more release-modifying agents, or by providing one or more passageways through the coating. The ratio of hydrophobic polymer to water soluble material is determined by, among other factors, the release rate required and the solubility characteristics of the materials selected. The release-modifying agents which function as pore-formers may be organic or inorganic, and include materials that can be dissolved, extracted or leached from the coating in the environment of use. The pore-formers may comprise one or more hydrophilic polymers such as hydroxypropylmethylcellulose. The controlled-release coatings can also include erosion-promoting agents such as starch and gums. The controlled-release coatings of the present invention can also include materials useful for making microporous lamina in the environment of use, such as polycarbonates comprised of linear polyesters of carbonic acid in which carbonate groups reoccur in the polymer chain.

In a preferred embodiment, the dosage form is multiparticulate wherein the individual particles (granules, pellets and the like) comprising a prolonged release coating are contained in a capsule, optionally together with additional excipients which may be contained in the capsule in powder form or also in form of particles (granules, pellets and the like). In the latter case, the capsules contain at least two different types of particles, namely particles that contain Tapentadol and particles that do not contain Tapentadol.

In another preferred embodiment, the dosage form is multiparticulate wherein the individual particles (granules, pellets and the like) comprising a prolonged release coating are contained in a tablet containing an extra-particulate material (multiple unit pellet system). The extra-particulate material preferably contains at least one excipient selected from binders, disintegrants and lubricants.

In preferred embodiments of the pharmaceutical dosage form according to the invention, Tapentadol is embedded in a prolonged release matrix.

Preferably, the pharmaceutical dosage form according to the invention, preferably the prolonged release matrix, contains at least one physiologically acceptable polymer that serves the purpose of retarding release of the pharmacologically active ingredient from the pharmaceutical dosage form. Thus, said at least one physiologically acceptable polymer is part of the prolonged release matrix of the pharmaceutical dosage form according to the invention.

Preferably, the prolonged release matrix comprises or essentially consists of at least one prolonged release matrix material selected from the group consisting of hydrophilic or hydrophobic polymers and hydrocarbons.

In preferred embodiments of the pharmaceutical dosage form according to the invention, the prolonged release matrix comprises or essentially consists of at least one polymer selected from the group consisting of
- polysaccharides or gums (e.g. xanthan gum, guar gum, karaya gum, locust bean gum, sodium alginate, carob gum, chitosan, polysaccharides of mannose and galactose, pectin, tragacanth, agar-agar);
- cellulose ethers (e.g. HPMC, HPC, HEC, MC, EC);
- cellulose esters (e.g. cellulose acetate, cellulose acetate succinate, cellulose acetate phthalate, cellulose acetate butyrate);
- polyalkylene glycols and polyalkylene oxides;
- polyvinylalcohol (PVA), cross-linked polyvinylalcohol (PVA), polyvinylpyrrolidone (PVP), cross-linked polyvinylpyrrolidone (PVP), polyvinylpyrrolidone-vinylacetate copolymers, polyvinylchloride (PVC), polyethylene vinyl acetate (PVAc), polydimethylsiloxane (PDS), polyether urethane (PEU), polylactic acid (PLA), polyglycolic acid (PGA), polycaprolactone (PCL), polyanhydrides, polyorthoesters;
- acrylic resins (e.g. cross-linked homopolymers and copolymers of acrylic acid, acrylic acid and methacrylic acid copolymers, methacrylic acid copolymers, methyl methacrylate copolymers, ethoxyethyl methacrylates, cynaoethyl methacrylate, methyl methacrylate, copolymers, methacrylic acid copolymers, methyl methacrylate copolymers, methyl methacrylate copolymers, methyl methacrylate copolymers, methacrylic acid copolymer, aminoalkyl methacrylate copolymer, methacrylic acid copolymers, methyl methacrylate copolymers, poly(acrylic acid), poly(methacrylic acid, methacrylic acid alkylamide copolymer, poly(methyl methacrylate), poly(methacrylic acid) (anhydride), methyl methacrylate, polymethacrylate, methyl methacrylate copolymer, poly(methyl methacrylate), poly(methyl methacrylate) copolymer, polyacrylamide, aminoalkyl methacrylate copolymer, poly(methacrylic acid anhydride), and glycidyl methacrylate copolymers, polyhydroxy ethyl methacrylate (PHEMA), polyacrylamide); and
- protein derived materials.

Preferably, the prolonged release matrix comprises or essentially consists of at least one hydrocarbon selected from the group consisting of long chain (C₈-C₅₀, especially C₁₂-C₄₀) fatty acids, long chain fatty alcohols, glyceryl esters of long chain fatty acids, mineral oils, vegetable oils, and waxes.

Preferably, the prolonged release matrix comprises or essentially consists of a prolonged release matrix material selected from the group consisting of (i) hydroxypropylmethylcellulose (HPMC); (ii) hydroxypropylcellulose (HPC); (iii) hydroxyethylcellulose (HEC); (iv) microcrystalline cellulose (MCC); (v) ethylcellulose (EC); (vi) polyvinyl acetate (PVAc); (vii) polyvinylpyrrolidone (PVP); (viii) polyvinylpyrrolidone-vinylacetate copolymer (PVP/PVAc); (ix) poly(ethyl acrylate-co-methyl methacrylate-co-trimethylammonioethyl methacrylate chloride); (x) poly(butyl methacrylate-co-(2-dimethylaminoethyl) methacrylate-co-methyl methacrylate); (xi) poly(methyl methacrylate-co-methacrylic acid); (xii) poly(ethyl acrylate-co-methacrylic acid); (xiii) poly(methyl acrylate-co-methyl methacrylate-co-methacrylic acid); (xiv) poly (ethyl acrylate-co-methyl methacrylate); (xv) poly(ethylene oxide) (PEO); (xvi) polyethylene glycol (PEG); (xvii) long chain fatty alcohol having 8 to 50 carbon atoms, preferably 12 to 40 carbon atoms, which may be saturated or unsaturated, linear or branched; (xviii) cetostearyl alcohol; (xix) stearyl alcohol; (xx) cetyl alcohol; (xxi) hydrocarbon selected from the group consisting of long chain fatty acids having 8 to 50 carbon atoms, preferably 12 to 40 carbon atoms, which may be saturated or unsaturated, linear or branched; glyceryl esters of such long chain fatty acids, mineral oils, vegetable oils, and waxes; (xxii) xanthan gum; (xxiii) sodium alginate; (xxiv) guar gum; (xxv) locust bean gum; and any mixtures of the foregoing. Preferably, the content of the prolonged release matrix material is within the range of 15±10 wt.-%, or 20±10 wt.-%, or 25±10 wt.-%, or 30±10 wt.-%, or 35±10 wt.-%, or 40±10 wt.-%, or 45±10 wt.-%, or 50±10 wt.-%, or 55±10 wt.-%, or 60±10 wt.-%, or 65±10 wt.-%, or 70±10 wt.-%, or 75±10 wt.-%, or 80±10 wt.-%, in each case relative to the total weight of the dosage form.

Preferably, the pharmaceutical dosage forms according to the invention comprise neither poly(alkylene oxide), e.g. poly(ethylene oxide), nor ethylene vinylacetate copolymers (EVA). For the purpose of the specification, poly(alkylene oxide) is distinguished from poly(alkylene glycol) by its molecular weight; polymers having a weight average molecular weight M_{w} of less than 100,000 g/mol are to be regarded as poly(alkylene glycol), whereas polymers having a weight average molecular weight M_{w} of 100,000 g/mol or more are to be regarded as poly(alkylene oxide).

Preferably, the prolonged release matrix comprises a cellulose derivative selected from cellulose ethers and cellulose esters or a poly(meth)acrylate or copolymer thereof.

Preferably, the cellulose derivative is a cellulose ether selected from the group consisting of methylcellulose, ethylcellulose, propylcellulose, hydroxyethylcellulose, hydroxypropylcellulose and hydroxypropylmethylcellulose; preferably hydroxypropylmethylcellulose.

Preferably, the cellulose derivative is hydroxypropylmethylcellulose. Preferably, the hydroxypropylmethylcellulose is selected from hypromellose type 1828, 2208, 2906 and 2910 according to USP having the following methoxyl content hydroxypropoxyl content:

| | methoxyl (%) | | hydroxypropoxyl (%) | |
|---|---|---|---|---|
| type | min | max | min | max |
| 1828 | 16.5 | 20.0 | 23.0 | 32.0 |
| 2208 | 19.0 | 24.0 | 4.0 | 12.0 |
| 2906 | 27.0 | 30.0 | 4.0 | 7.5 |
| 2910 | 28.0 | 30.0 | 7.0 | 12.0 |

Preferably, the viscosity of the physiologically acceptable polymer, preferably cellulose derivative, more preferably hydroxypropylmethylcellulose, is within the range of 100,000±80,000 mPa·s, more preferably 100,000±60,000 mPa·s, still more preferably 100,000±40,000 mPa·s, yet more preferably 100,000±20,000 mPa·s.

Preferably, the number average molecular weight Mₙ of the physiologically acceptable polymer, preferably cellulose derivative, more preferably hydroxypropylmethylcellulose, is not more than 220,000 g/mol, more preferably not more than 180,000 g/mol, still more preferably not more than 140,000 g/mol, yet more preferably not more than 120,000 g/mol, even more preferably not more than 110,000 g/mol, most preferably not more than 86,000 g/mol, and in particular not more than 63,000 g/mol.

Preferably, the weight content of the physiologically acceptable polymer, preferably cellulose ether, is at least 2.0 wt.-%, more preferably at least 3.0 wt.-%, still more preferably at least 4.0 wt.-%, yet more preferably at least 5.0 wt.-%, even more preferably at least 6.0 wt.-%, most preferably at least 7.0 wt.-%, and in particular at least 8.0 wt.-%, in each case relative to the total weight of the pharmaceutical dosage form.

Preferably, the weight content of the physiologically acceptable polymer, preferably cellulose ether, is at most 62.5 wt.-%, more preferably at most 60 wt.-%, still more preferably at most 57.5 wt.-%, yet more preferably at most 55 wt.-%, even more preferably at most 52.5 wt.-%, most preferably at most 50 wt.-%, and in particular at most 47.5 wt.-%, in each case relative to the total weight of the pharmaceutical dosage form.

Preferably, the weight content of the physiologically acceptable polymer, preferably cellulose ether, is within the range of from 30±28 wt.-%, more preferably 30±26 wt.-%, still more preferably 30±24 wt.-%, yet more preferably 30±22 wt.-%, even more preferably 30±20 wt.-%, most preferably 30±18 wt.-%, and in particular 30±16 wt.-%, in each case relative to the total weight of the pharmaceutical dosage form.

When the pharmaceutical dosage form contains more than one physiologically acceptable polymer serving the purpose of significantly retarding release of the pharmacologically active ingredient from the pharmaceutical dosage form, preferably cellulose ether, the above percentages refer to the total content of all such physiologically acceptable polymers, preferably cellulose ethers, that are contained in the pharmaceutical dosage form.

In a particularly preferred embodiment, the pharmaceutical dosage form according to the invention comprises a weight equivalent dose of Tapentadol within the range of from 10 to 300 mg, e.g. 25 mg, 50 mg, 100 mg, 150 mg, 200 mg, or 250 mg, in each case based on the free base of Tapentadol, and a prolonged release matrix in which the salt of Tapentadol with phosphoric acid, preferably the dihydrogenphosphate salt of Tapentadol, is embedded; wherein the prolonged release matrix comprises as prolonged release matrix material hydroxypropylmethylcellulose (HPMC) preferably in an amount of from 5.0 to 60 wt.-%, e.g. 15±10 wt.-%, or 20±10 wt.-%, or 25±10 wt.-%, or 30±10 wt.-%, or 35±10 wt.-%, or 40±10 wt.-%, or 45±10 wt.-%, or 50±10 wt.-%,in each case relative to the total weight of the dosage form.

In a particularly preferred embodiment, the pharmaceutical dosage form according to the invention comprises a weight equivalent dose of Tapentadol within the range of from 10 to 300 mg, e.g. 25 mg, 50 mg, 100 mg, 150 mg, 200 mg, or 250 mg, in each case based on the free base of Tapentadol, and a prolonged release matrix in which the salt of Tapentadol with phosphoric acid, preferably the dihydrogenphosphate salt of Tapentadol, is embedded; wherein the prolonged release matrix comprises as prolonged release matrix material hydroxypropylcellulose (HPC) preferably in an amount of from 10 to 50 wt.-%, e.g. 20±10 wt.-%, or 25±10 wt.-%, or 30±10 wt.-%, or 35±10 wt.-%, or 40±10 wt.-%, in each case relative to the total weight of the dosage form.

In a particularly preferred embodiment, the pharmaceutical dosage form according to the invention comprises a weight equivalent dose of Tapentadol within the range of from 10 to 300 mg, e.g. 25 mg, 50 mg, 100 mg, 150 mg, 200 mg, or 250 mg, in each case based on the free base of Tapentadol, and a prolonged release matrix in which the salt of Tapentadol with phosphoric acid, preferably the dihydrogenphosphate salt of Tapentadol, is embedded; wherein the prolonged release matrix comprises as prolonged release matrix material hydroxyethylcellulose (HEC) preferably in an amount of from 5.0 to 50 wt.-%, e.g. 15±10 wt.-%, or 20±10 wt.-%, or 25±10 wt.-%, or 30±10 wt.-%, or 35±10 wt.-%, or 40±10 wt.-%, in each case relative to the total weight of the dosage form.

In a particularly preferred embodiment, the pharmaceutical dosage form according to the invention comprises a weight equivalent dose of Tapentadol within the range of from 10 to 300 mg, e.g. 25 mg, 50 mg, 100 mg, 150 mg, 200 mg, or 250 mg, in each case based on the free base of Tapentadol, and a prolonged release matrix in which the salt of Tapentadol with phosphoric acid, preferably the dihydrogenphosphate salt of Tapentadol, is embedded; wherein the prolonged release matrix comprises as prolonged release matrix material microcrystalline cellulose (MCC) preferably in an amount of from 10 to 70 wt.-%, e.g. 20±10 wt.-%, or 25±10 wt.-%, or 30±10 wt.-%, or 35±10 wt.-%, or 40±10 wt.-%, or 45±10 wt.-%, or 50±10 wt.-%, or 55±10 wt.-%, or 60±10 wt.-%, in each case relative to the total weight of the dosage form.

In a particularly preferred embodiment, the pharmaceutical dosage form according to the invention comprises a weight equivalent dose of Tapentadol within the range of from 10 to 300 mg, e.g. 25 mg, 50 mg, 100 mg, 150 mg, 200 mg, or 250 mg, in each case based on the free base of Tapentadol, and a prolonged release matrix in which the salt of Tapentadol with phosphoric acid, preferably the dihydrogenphosphate salt of Tapentadol, is embedded; wherein the prolonged release matrix comprises as prolonged release matrix material ethylcellulose (EC) preferably in an amount of from 5.0 to 30 wt.-%, e.g. 15±10 wt.-%, or 20±10 wt.-%, in each case relative to the total weight of the dosage form.

In a particularly preferred embodiment, the pharmaceutical dosage form according to the invention comprises a weight equivalent dose of Tapentadol within the range of from 10 to 300 mg, e.g. 25 mg, 50 mg, 100 mg, 150 mg, 200 mg, or 250 mg, in each case based on the free base of Tapentadol, and a prolonged release matrix in which the salt of Tapentadol with phosphoric acid, preferably the dihydrogenphosphate salt of Tapentadol, is embedded; wherein the prolonged release matrix comprises as prolonged release matrix material polyvinyl acetate (PVAc) preferably in an amount of from 25 to 70 wt.-%, e.g. 35±10 wt.-%, or 40±10 wt.-%, or 45±10 wt.-%, or 50±10 wt.-%, or 55±10 wt.-%, or 60±10 wt.-%, in each case relative to the total weight of the dosage form. Preferably, the polyvinyl acetate is employed in form of a blend with polyvinylpyrrolidone (povidone). Such a preferred blend is commercially available e.g. as Kollidon® SR (80 wt.-% polyvinyl acetate, 19 wt.-% povidone, 0.8 wt.-% sodium lauryl sulfate, and 0.2 wt.-% silicic acid).

In a particularly preferred embodiment, the pharmaceutical dosage form according to the invention comprises a weight equivalent dose of Tapentadol within the range of from 10 to 300 mg, e.g. 25 mg, 50 mg, 100 mg, 150 mg, 200 mg, or 250 mg, in each case based on the free base of Tapentadol, and a prolonged release matrix in which the salt of Tapentadol with phosphoric acid, preferably the dihydrogenphosphate salt of Tapentadol, is embedded; wherein the prolonged release matrix comprises as prolonged release matrix material polyvinylpyrrolidone (PVP) preferably in an amount of from 2.0 to 21 wt.-%, e.g. 10±5.0 wt.-%, or 15±5.0 wt.-%, in each case relative to the total weight of the dosage form.

In a particularly preferred embodiment, the pharmaceutical dosage form according to the invention comprises a weight equivalent dose of Tapentadol within the range of from 10 to 300 mg, e.g. 25 mg, 50 mg, 100 mg, 150 mg, 200 mg, or 250 mg, in each case based on the free base of Tapentadol, and a prolonged release matrix in which the salt of Tapentadol with phosphoric acid, preferably the dihydrogenphosphate salt of Tapentadol, is embedded; wherein the prolonged release matrix comprises as prolonged release matrix material polyvinylpyrrolidone-vinylacetate copolymer (PVP/PVAc) preferably in an amount of from 1.0 to 30 wt.-%, e.g. 15±10 wt.-%, or 20±10 wt.-%, in each case relative to the total weight of the dosage form.

In a particularly preferred embodiment, the pharmaceutical dosage form according to the invention comprises a weight equivalent dose of Tapentadol within the range of from 10 to 300 mg, e.g. 25 mg, 50 mg, 100 mg, 150 mg, 200 mg, or 250 mg, in each case based on the free base of Tapentadol, and a prolonged release matrix in which the salt of Tapentadol with phosphoric acid, preferably the dihydrogenphosphate salt of Tapentadol, is embedded; wherein the prolonged release matrix comprises as prolonged release matrix material poly(ethyl acrylate-co-methyl methacrylate-co-trimethyl-ammonioethyl methacrylate chloride) preferably in an amount of from 5.0 to 45 wt.-%, e.g. 15±10 wt.-%, or 20±10 wt.-%, or 25±10 wt.-%, or 30±10 wt.-%, or 35±10 wt.-%, in each case relative to the total weight of the dosage form. Poly(ethyl acrylate-co-methyl methacrylate-co-trimethylammonioethyl methacrylate chloride) is commercially available e.g. as Eudragit® RS and Eudragit® RL.

In a particularly preferred embodiment, the pharmaceutical dosage form according to the invention comprises a weight equivalent dose of Tapentadol within the range of from 10 to 300 mg, e.g. 25 mg, 50 mg, 100 mg, 150 mg, 200 mg, or 250 mg, in each case based on the free base of Tapentadol, and a prolonged release matrix in which the salt of Tapentadol with phosphoric acid, preferably the dihydrogenphosphate salt of Tapentadol, is embedded; wherein the prolonged release matrix comprises as prolonged release matrix material poly(butyl methacrylate-co-(2-dimethylaminoethyl) methacrylate-co-methyl methacrylate) preferably in an amount of from 5.0 to 45 wt.-%, e.g. 15±10 wt.-%, or 20±10 wt.-%, or 25±10 wt.-%, or 30±10 wt.-%, or 35±10 wt.-%, in each case relative to the total weight of the dosage form. Poly(butyl methacrylate-co-(2-dimethylaminoethyl) methacrylate-co-methyl methacrylate) is commercially available e.g. as Eudragit® E.

In a particularly preferred embodiment, the pharmaceutical dosage form according to the invention comprises a weight equivalent dose of Tapentadol within the range of from 10 to 300 mg, e.g. 25 mg, 50 mg, 100 mg, 150 mg, 200 mg, or 250 mg, in each case based on the free base of Tapentadol, and a prolonged release matrix in which the salt of Tapentadol with phosphoric acid, preferably the dihydrogenphosphate salt of Tapentadol, is embedded; wherein the prolonged release matrix comprises as prolonged release matrix material poly(methyl methacrylate-co-methacrylic acid) preferably in an amount of from 5.0 to 45 wt.-%, e.g. 15±10 wt.-%, or 20±10 wt.-%, or 25±10 wt.-%, or 30±10 wt.-%, or 35±10 wt.-%, in each case relative to the total weight of the dosage form. Poly(methyl methacrylate-co-methacrylic acid) is commercially available e.g. as Eudragit® L.

In a particularly preferred embodiment, the pharmaceutical dosage form according to the invention comprises a weight equivalent dose of Tapentadol within the range of from 10 to 300 mg, e.g. 25 mg, 50 mg, 100 mg, 150 mg, 200 mg, or 250 mg, in each case based on the free base of Tapentadol, and a prolonged release matrix in which the salt of Tapentadol with phosphoric acid, preferably the dihydrogenphosphate salt of Tapentadol, is embedded; wherein the prolonged release matrix comprises as prolonged release matrix material poly(ethyl acrylate-co-methacrylic acid) preferably in an amount of from 5.0 to 45 wt.-%, e.g. 15±10 wt.-%, or 20±10 wt.-%, or 25±10 wt.-%, or 30±10 wt.-%, or 35±10 wt.-%, in each case relative to the total weight of the dosage form. Poly(ethyl acrylate-co-methacrylic acid) is commercially available e.g. as Eudragit® S.

In a particularly preferred embodiment, the pharmaceutical dosage form according to the invention comprises a weight equivalent dose of Tapentadol within the range of from 10 to 300 mg, e.g. 25 mg, 50 mg, 100 mg, 150 mg, 200 mg, or 250 mg, in each case based on the free base of Tapentadol, and a prolonged release matrix in which the salt of Tapentadol with phosphoric acid, preferably the dihydrogenphosphate salt of Tapentadol, is embedded; wherein the prolonged release matrix comprises as prolonged release matrix material poly(methyl acrylate-co-methyl methacrylate-co-methacrylic acid) preferably in an amount of from 5.0 to 45 wt.-%, e.g. 15±10 wt.-%, or 20±10 wt.-%, or 25±10 wt.-%, or 30±10 wt.-%, or 35±10 wt.-%, in each case relative to the total weight of the dosage form. Poly(methyl acrylate-co-methyl methacrylate-co-methacrylic acid) is commercially available e.g. as Eudragit® FS.

In a particularly preferred embodiment, the pharmaceutical dosage form according to the invention comprises a weight equivalent dose of Tapentadol within the range of from 10 to 300 mg, e.g. 25 mg, 50 mg, 100 mg, 150 mg, 200 mg, or 250 mg, in each case based on the free base of Tapentadol, and a prolonged release matrix in which the salt of Tapentadol with phosphoric acid, preferably the dihydrogenphosphate salt of Tapentadol, is embedded; wherein the prolonged release matrix comprises as prolonged release matrix material poly(ethyl acrylate-co-methyl methacrylate) preferably in an amount of from 5.0 to 45 wt.-%, e.g. 15±10 wt.-%, or 20±10 wt.-%, or 25±10 wt.-%, or 30±10 wt.-%, or 35±10 wt.-%, in each case relative to the total weight of the dosage form. Poly(ethyl acrylate-co-methyl methacrylate) is commercially available e.g. as Eudragit® NE.

In a particularly preferred embodiment, the pharmaceutical dosage form according to the invention comprises a weight equivalent dose of Tapentadol within the range of from 10 to 300 mg, e.g. 25 mg, 50 mg, 100 mg, 150 mg, 200 mg, or 250 mg, in each case based on the free base of Tapentadol, and a prolonged release matrix in which the salt of Tapentadol with phosphoric acid, preferably the dihydrogenphosphate salt of Tapentadol, is embedded; wherein the prolonged release matrix comprises as prolonged release matrix material poly(ethylene oxide) (PEO) preferably in an amount of from 25 to 65 wt.-%, e.g. 35±10 wt.-%, or 40±10 wt.-%, or 45±10 wt.-%, or 50±10 wt.-%, or 55±10 wt.-%, in each case relative to the total weight of the dosage form.

In a particularly preferred embodiment, the pharmaceutical dosage form according to the invention comprises a weight equivalent dose of Tapentadol within the range of from 10 to 300 mg, e.g. 25 mg, 50 mg, 100 mg, 150 mg, 200 mg, or 250 mg, in each case based on the free base of Tapentadol, and a prolonged release matrix in which the salt of Tapentadol with phosphoric acid, preferably the dihydrogenphosphate salt of Tapentadol, is embedded; wherein the prolonged release matrix comprises as prolonged release matrix material polyethylene glycol (PEG) preferably in an amount of from 5.0 to 35 wt.-%, e.g. 15±10 wt.-%, or 20±10 wt.-%, or 25±10 wt.-%, in each case relative to the total weight of the dosage form.

In a particularly preferred embodiment, the pharmaceutical dosage form according to the invention comprises a weight equivalent dose of Tapentadol within the range of from 10 to 300 mg, e.g. 25 mg, 50 mg, 100 mg, 150 mg, 200 mg, or 250 mg, in each case based on the free base of Tapentadol, and a prolonged release matrix in which the salt of Tapentadol with phosphoric acid, preferably the dihydrogenphosphate salt of Tapentadol, is embedded; wherein the prolonged release matrix comprises as prolonged release matrix material a long chain fatty alcohol having 8 to 50 carbon atoms, preferably 12 to 40 carbon atoms, which may be saturated or unsaturated, linear or branched, preferably in an amount of from 15 to 40 wt.-%, e.g. 25±10 wt.-%, or 30±10 wt.-%, in each case relative to the total weight of the dosage form.

In a particularly preferred embodiment, the pharmaceutical dosage form according to the invention comprises a weight equivalent dose of Tapentadol within the range of from 10 to 300 mg, e.g. 25 mg, 50 mg, 100 mg, 150 mg, 200 mg, or 250 mg, in each case based on the free base of Tapentadol, and a prolonged release matrix in which the salt of Tapentadol with phosphoric acid, preferably the dihydrogenphosphate salt of Tapentadol, is embedded; wherein the prolonged release matrix comprises as prolonged release matrix material cetostearyl alcohol preferably in an amount of from 15 to 40 wt.-%, e.g. 25±10 wt.-%, or 30±10 wt.-%, in each case relative to the total weight of the dosage form.

In a particularly preferred embodiment, the pharmaceutical dosage form according to the invention comprises a weight equivalent dose of Tapentadol within the range of from 10 to 300 mg, e.g. 25 mg, 50 mg, 100 mg, 150 mg, 200 mg, or 250 mg, in each case based on the free base of Tapentadol, and a prolonged release matrix in which the salt of Tapentadol with phosphoric acid, preferably the dihydrogenphosphate salt of Tapentadol, is embedded; wherein the prolonged release matrix comprises as prolonged release matrix material stearyl alcohol preferably in an amount of from 15 to 40 wt.-%, e.g. 25±10 wt.-%, or 30±10 wt.-%, in each case relative to the total weight of the dosage form.

In a particularly preferred embodiment, the pharmaceutical dosage form according to the invention comprises a weight equivalent dose of Tapentadol within the range of from 10 to 300 mg, e.g. 25 mg, 50 mg, 100 mg, 150 mg, 200 mg, or 250 mg, in each case based on the free base of Tapentadol, and a prolonged release matrix in which the salt of Tapentadol with phosphoric acid, preferably the dihydrogenphosphate salt of Tapentadol, is embedded; wherein the prolonged release matrix comprises as prolonged release matrix material cetyl alcohol preferably in an amount of from 15 to 40 wt.-%, e.g. 25±10 wt.-%, or 30±10 wt.-%, in each case relative to the total weight of the dosage form.

In a particularly preferred embodiment, the pharmaceutical dosage form according to the invention comprises a weight equivalent dose of Tapentadol within the range of from 10 to 300 mg, e.g. 25 mg, 50 mg, 100 mg, 150 mg, 200 mg, or 250 mg, in each case based on the free base of Tapentadol, and a prolonged release matrix in which the salt of Tapentadol with phosphoric acid, preferably the dihydrogenphosphate salt of Tapentadol, is embedded; wherein the prolonged release matrix comprises as prolonged release matrix material a hydrocarbon selected from the group consisting of long chain fatty acids having 8 to 50 carbon atoms, preferably 12 to 40 carbon atoms, which may be saturated or unsaturated, linear or branched; glyceryl esters of such long chain fatty acids, mineral oils, vegetable oils, and waxes; in each case preferably in an amount of from 5.0 to 70 wt.-%, e.g. 15±10 wt.-%, or 20±10 wt.-%, or 25±10 wt.-%, or 30±10 wt.-%, or 35±10 wt.-%, or 40±10 wt.-%, or 45±10 wt.-%, or 50±10 wt.-%, or 55±10 wt.-%, or 60±10 wt.-%, in each case relative to the total weight of the dosage form.

In a particularly preferred embodiment, the pharmaceutical dosage form according to the invention comprises a weight equivalent dose of Tapentadol within the range of from 10 to 300 mg, e.g. 25 mg, 50 mg, 100 mg, 150 mg, 200 mg, or 250 mg, in each case based on the free base of Tapentadol, and a prolonged release matrix in which the salt of Tapentadol with phosphoric acid, preferably the dihydrogenphosphate salt of Tapentadol, is embedded; wherein the prolonged release matrix comprises as prolonged release matrix material xanthan gum preferably in an amount of from 15 to 40 wt.-%, e.g. 25±10 wt.-%, or 30±10 wt.-%, in each case relative to the total weight of the dosage form.

In a particularly preferred embodiment, the pharmaceutical dosage form according to the invention comprises a weight equivalent dose of Tapentadol within the range of from 10 to 300 mg, e.g. 25 mg, 50 mg, 100 mg, 150 mg, 200 mg, or 250 mg, in each case based on the free base of Tapentadol, and a prolonged release matrix in which the salt of Tapentadol with phosphoric acid, preferably the dihydrogenphosphate salt of Tapentadol, is embedded; wherein the prolonged release matrix comprises as prolonged release matrix material sodium alginate preferably in an amount of from 15 to 40 wt.-%, e.g. 25±10 wt.-%, or 30±10 wt.-%, in each case relative to the total weight of the dosage form.

In a particularly preferred embodiment, the pharmaceutical dosage form according to the invention comprises a weight equivalent dose of Tapentadol within the range of from 10 to 300 mg, e.g. 25 mg, 50 mg, 100 mg, 150 mg, 200 mg, or 250 mg, in each case based on the free base of Tapentadol, and a prolonged release matrix in which the salt of Tapentadol with phosphoric acid, preferably the dihydrogenphosphate salt of Tapentadol, is embedded; wherein the prolonged release matrix comprises as prolonged release matrix material guar gum preferably in an amount of from 5.0 to 35 wt.-%, e.g. 15±10 wt.-%, or 20±10 wt.-%, or 25±10 wt.-%, in each case relative to the total weight of the dosage form.

In a particularly preferred embodiment, the pharmaceutical dosage form according to the invention comprises a weight equivalent dose of Tapentadol within the range of from 10 to 300 mg, e.g. 25 mg, 50 mg, 100 mg, 150 mg, 200 mg, or 250 mg, in each case based on the free base of Tapentadol, and a prolonged release matrix in which the salt of Tapentadol with phosphoric acid, preferably the dihydrogenphosphate salt of Tapentadol, is embedded; wherein the prolonged release matrix comprises as prolonged release matrix material locust bean gum preferably in an amount of from 5.0 to 35 wt.-%, e.g. 15±10 wt.-%, or 20±10 wt.-%, or 25±10 wt.-%, in each case relative to the total weight of the dosage form.

In addition to the above ingredients, a prolonged release matrix may also contain suitable quantities of other materials, e.g. diluents, lubricants, binders, granulating aids, colorants, flavors and glidants that are conventional in the pharmaceutical art.

Pharmaceutical dosage forms containing prolonged release matrices in which Tapentadol is embedded may be prepared by conventional techniques that are known to the skilled person such as blending and direct compression, dry granulation, wet granulation, extrusion, and the like.

In a preferred embodiment, the pharmaceutical dosage form according to the invention is a capsule, preferably containing a plurality of particles containing a prolonged release coating.

In another preferred embodiment, the pharmaceutical dosage form according to the invention is a tablet. Preferably, the tablet is monolithic. Monolithic tablets according to the invention mean tablets that are optionally film coated, wherein the core of the tablets contains a compressed powder and/or granulate mixture. In particular (i), tablets prepared by direct compression of powder mixtures, (ii) tablets prepared by compression of mixtures comprising granules obtained by dry granulation and optionally extragranular excipients, (iii) tablets prepared by compression of mixtures comprising granules obtained by wet granulation and optionally extragranular excipients, and (iv) tablets prepared by compression of mixtures comprising granules obtained by extrusion granulation and optionally extragranular excipients are all to be regarded as monolithic tablets in accordance with the invention. However, multiple unit pellet systems or other dosage forms where a multitude of particles of specific design, weight and shape is mixed with an outer matrix material and subsequently compressed into tablets where the outer matrix material forms a continuous phase in which the pellets or particles are embedded, are preferably not to be regarded as monolithic tablets. Of course, capsules filled with a multitude of loose particles are not to be regarded as monolithic either.

Preferably, the tablet has a breaking strength of at least 100 N, preferably at least 150 N, more preferably at least 200 N. The breaking strength is preferably determined in accordance with Ph. Eur. 10, chapter 2.9.8. *"Resistance to Crushing of Tablets".*

Another aspect of the invention related to a method for preparing a pharmaceutical dosage form according to the invention as described above.

When the pharmaceutical dosage form contains particles comprising a prolonged release coating, in a preferred embodiment the method for the preparation according to the invention comprises the steps of
(A) providing inert starter pellets, e.g. nonpareils, which contain one or more excipients but no Tapentadol;
(B) providing a solution or dispersion of Tapentadol (including the salt with phosphoric acid) in water or an organic solvent or a mixture thereof, optionally together with the one or more excipients, wherein the organic solvent is preferably selected from ethanol and acetone;
(C) coating the inert starter pellets provided in step (A) with the solution or dispersion of Tapentadol provided in step (B), preferably in a fluidized bed, thereby obtaining intermediate particles containing an inert core not containing any Tapentadol and a drug coating layer encapsulating said core and comprising essentially the total amount of Tapentadol to be contained in the dosage form, optionally together with the one or more excipients;
(D) optionally, drying the intermediate particles obtained in step (C) thereby obtaining dried intermediate particles;
(E) providing a solution or dispersion of a prolonged release coating material in water or an organic solvent or a mixture thereof, optionally together with the one or more excipients, wherein the organic solvent is preferably selected from ethanol and acetone;
(F) coating the intermediate particles obtained in step (C) or the dried intermediate particles obtained in step (D) with the solution or dispersion of prolonged release coating material provided in step (E), preferably in a fluidized bed, thereby obtaining prolonged release particles containing an inert core not containing any Tapentadol, a drug coating layer encapsulating said core and comprising essentially the total amount of Tapentadol, optionally together with the one or more excipients, and a prolonged release coating encapsulating said core and said drug coating layer;
(G) optionally, drying the prolonged release particles obtained in step (F) thereby obtained dried prolonged release particles; and
(H) either filling the prolonged release particles obtained in step (F) or the dried prolonged release particles obtained in step (G) into capsules; or mixing the prolonged release particles obtained in step (F) or the dried prolonged release particles obtained in step (G) with extra-particle excipients and compressing the mixture into tablets (multiple unit pellet systems).

Another aspect of the invention relates to a pharmaceutical dosage form that is obtainable by this preferred method according to the invention as described above.

When the pharmaceutical dosage form contains particles comprising a prolonged release coating, in a preferred embodiment the method for the preparation according to the invention comprises the steps of
(A) providing a mixture containing essentially the total amount of Tapentadol (including the salt with phosphoric acid) to be contained in the dosage form, optionally together with one or more excipients;
(B) preparing drug pellets from the mixture provided in step (A) by dry granulation, wet granulation or extrusion, wherein wet granulation preferably involved use of a solvent selected from water, ethanol, acetone, and any mixture thereof;
(C) optionally, drying and/or spheronizing the drug pellets prepared in step (B) thereby obtaining dried and/or spheronized drug pellets;
(D) providing a solution or dispersion of a prolonged release coating material in water or an organic solvent or a mixture thereof, optionally together with the one or more excipients, wherein the organic solvent is preferably selected from ethanol and acetone;
(E) coating the drug pellets prepared in step (B) or the dried and/or spheronized drug pellets obtained in step (C) with the solution or dispersion of prolonged release coating material provided in step (D), preferably in a fluidized bed, thereby obtaining prolonged release particles containing a core comprising essentially the total amount of Tapentadol, optionally together with one or more excipients, and a prolonged release coating encapsulating said core;
(F) optionally, drying the prolonged release particles obtained in step (E) thereby obtained dried prolonged release particles; and
(G) either filling the prolonged release particles obtained in step (E) or the dried prolonged release particles obtained in step (F) into capsules; or mixing the prolonged release particles obtained in step (E) or the dried prolonged release particles obtained in step (F) with extra-particle excipients and compressing the mixture into tablets (multiple unit pellet systems).

Another aspect of the invention relates to a pharmaceutical dosage form that is obtainable by this preferred method according to the invention as described above.

When the pharmaceutical dosage form contains a prolonged release matrix in which the Tapentadol (including the salt with phosphoric acid) is embedded, in a preferred embodiment the method for the preparation according to the invention comprises the steps of
(a) providing a mixture containing essentially the total amount of Tapentadol (including the salt with phosphoric acid) to be contained in the dosage form and at least one prolonged release matrix material, optionally together with one or more excipients;
(b) optionally granulating the mixture provided in step (a) thereby obtaining a granulate, wherein granulating preferably involves: (i) wet granulating by means of a solvent, preferably selected from water, ethanol, acetone, and any mixture thereof, optionally followed by drying; (ii) dry granulation; or (iii) extrusion;
(c) optionally mixing the granulate obtained in step (b) with one or more excipients thereby obtaining a granulate mixture;
(d) compressing the mixture provided in step (a) or the granulate obtained in step (b) or the granulate mixture obtained in step (c) into tablets;
(e) optionally, film coating the tablets compressed in step (d).

Preferably, compressing in step (d) of the process according to the invention is performed at a compression force of not more than 20 kN, more preferably not more than 15 kN, still more preferably not more than 10 kN, yet more preferably not more than 9.5 kN, even more preferably not more than 9.0 kN, most preferably not more than 8.75 kN, and in particular not more than 8.5 kN.

Preferably, compressing in step (d) of the process according to the invention is performed under conditions such that the compressed tablet has a breaking strength of at least 100 N, more preferably at least 150 N, still more preferably at least 200 N.

Preferably, the pharmaceutical dosage form according to the invention is not prepared by thermoforming such as hot-melt extrusion.

Preferably, the pharmaceutical dosage form according to the invention does not contain a multitude of particles or pellets of specific design, shape and weight that are optionally compressed into tablets wherein said particles or pellets form a discontinuous phase within a continuous phase of an outer matrix material.

Preferably, the pharmaceutical dosage form according to the invention does not contain an opioid antagonist. Opioid antagonists are entities that modify the response of opioid receptors. Opioid antagonists include Naloxone, Naltrexone, Diprenorphine, Etorphine, Dihydroetorphine, Nalinefene, Cyclazacine, Levallorphan, pharmaceutically acceptable salts thereof and mixtures thereof.

Another aspect of the invention relates to the pharmaceutical dosage form according to the invention as described above for use in the treatment of pain, wherein the dosage form is orally administered, preferably twice daily.

Another aspect of the invention relates to the use of a salt of Tapentadol with phosphoric acid, preferably the dihydrogenphosphate salt of Tapentadol, for the manufacture of a pharmaceutical dosage form according to the invention as described above for the treatment of pain, wherein the dosage form is orally administered, preferably twice daily.

Another aspect of the invention relates to a method of treating pain comprising the step of administering to a subject in need thereof orally, preferably twice daily, a pharmaceutical dosage form according to the invention as described above.

Preferably, the pain is chronic pain.

In preferred embodiments, the pharmaceutical dosage form according to the invention provides in a patient population of at least 10 patients, preferably at least 50 patients, an average value of Tₘₐₓ within the range of 5.0±3.0 hours after oral administration.

In preferred embodiments of the pharmaceutical dosage form according to the invention, the weight equivalent dose of Tapentadol that is contained in the pharmaceutical dosage form amounts to 50 mg based on the free base of Tapentadol and wherein the dosage form after oral administration in a patient population of at least 50 patients provides an average value of
- Cₘₐₓ within the range of 12±3 ng/ml; and/or
- AUCₗₐₛₜ within the range of 204±50 ng·h/ml; and/or
- AUC_{∞} within the range of 214±50 ng·h/ml.

In preferred embodiments of the pharmaceutical dosage form according to the invention, the weight equivalent dose of Tapentadol that is contained in the pharmaceutical dosage form amounts to 100 mg based on the free base of Tapentadol and wherein the dosage form after oral administration in a patient population of at least 50 patients provides an average value of
- Cₘₐₓ within the range of 29±6 ng/ml; and/or
- AUCₗₐₛₜ within the range of 440±100 ng·h/ml; and/or
- AUC_{∞} within the range of 447±100 ng·h/ml.

In preferred embodiments of the pharmaceutical dosage form according to the invention, the weight equivalent dose of Tapentadol that is contained in the pharmaceutical dosage form amounts to 150 mg based on the free base of Tapentadol and wherein the dosage form after oral administration in a patient population of at least 50 patients provides an average value of
- Cₘₐₓ within the range of 47±9 ng/ml; and/or
- AUCₗₐₛₜ within the range of 662±150 ng·h/ml; and/or
- AUC_{∞} within the range of 665±150 ng·h/ml.

In preferred embodiments of the pharmaceutical dosage form according to the invention, the weight equivalent dose of Tapentadol that is contained in the pharmaceutical dosage form amounts to 200 mg based on the free base of Tapentadol and wherein the dosage form after oral administration in a patient population of at least 50 patients provides an average value of
- Cₘₐₓ within the range of 64±12 ng/ml; and/or
- AUCₗₐₛₜ within the range of 890±200 ng·h/ml; and/or
- AUC_{∞} within the range of 895±200 ng·h/ml.

In preferred embodiments of the pharmaceutical dosage form according to the invention, the weight equivalent dose of Tapentadol that is contained in the pharmaceutical dosage form amounts to 250 mg based on the free base of Tapentadol and wherein the dosage form after oral administration in a patient population of at least 50 patients provides an average value of
- Cₘₐₓ within the range of 85±15 ng/ml; and/or
- AUCₗₐₛₜ within the range of 1141±250 ng·h/ml; and/or
- AUC_{∞} within the range of 1145±250 ng·h/ml.

Particularly preferred embodiments of the invention are summarized as clauses 1 to 67 hereinafter:
Clause 1: A pharmaceutical dosage form comprising Tapentadol for oral administration twice daily;
   wherein Tapentadol is present as a salt with phosphoric acid; wherein the dosage form provides prolonged release of Tapentadol; and wherein the weight equivalent dose of Tapentadol that is contained in the pharmaceutical dosage form is within the range of from 10 to 300 mg based on the free base of Tapentadol.
Clause 2: The pharmaceutical dosage form according to clause 1, wherein the salt is the dihydrogenphosphate salt of Tapentadol, a solvate, an ansolvate, and/or a polymorph thereof.
Clause 3: The pharmaceutical dosage form according to clause 1 or 2, which comprises one, two or more physiologically acceptable excipients.
Clause 4: The pharmaceutical dosage form according to any of the preceding clauses, which after oral administration provides plasma levels of Tapentadol providing pain relief for a duration of at least 6 hours.
Clause 5: The pharmaceutical dosage form according to any of the preceding clauses, which provides an in vitro dissolution profile, when measured by the USP Paddle Method at 50 rpm in 900 ml aqueous phosphate buffer at pH 6.8 at 37° C, wherein after 0.5 hour 20±20 wt.-%; preferably 20±15 wt.-%; more preferably 20±10 wt.-%; after 4 hours 60±20 wt.-%; preferably 60±15 wt.-%; more preferably 60±10 wt.-%; and after 12 hours at least 60 wt.-%; preferably at least 70 wt.-%; more preferably at least 80 wt.-% of the Tapentadol that was originally contained in the dosage form have been released.
Clause 6: The pharmaceutical dosage form according to any of the preceding clauses, which provides an in vitro dissolution profile, when measured by the USP Paddle Method at 50 rpm in 900 ml aqueous phosphate buffer at pH 6.8 at 37° C, wherein - after 1 hour 25±15 wt.-%;- after 2 hours 35±20 wt.-%;- after 4 hours 50±20 wt.-%;- after 8 hours 80±20 wt.-%; of the Tapentadol that was originally contained in the dosage form have been released.
Clause 7: The pharmaceutical dosage form according to any of the preceding clauses, which provides an in vitro dissolution profile, when measured by the USP Paddle Method at 50 rpm in 900 ml aqueous buffer at pH 4.5 at 37° C, wherein after 0.5 hour 20±20 wt.-%; preferably 20±15 wt.-%; more preferably 20±10 wt.-%; after 4 hours 60±20 wt.-%; preferably 60±15 wt.-%; more preferably 60±10 wt.-%; and after 12 hours at least 60 wt.-%; preferably at least 70 wt.-%; more preferably at least 80 wt.-%
   of the Tapentadol that was originally contained in the dosage form have been released.
Clause 8: The pharmaceutical dosage form according to any of the preceding clauses, which provides an in vitro dissolution profile, when measured by the USP Paddle Method at 50 rpm in 900 ml aqueous buffer at pH 4.5 at 37° C, wherein - after 1 hour 25±15 wt.-%;- after 2 hours 35±20 wt.-%;-after 4 hours 50±20 wt.-%;- after 8 hours 80±20 wt.-%; of the Tapentadol that was originally contained in the dosage form have been released.
Clause 9: The pharmaceutical dosage form according to any of the preceding clauses, which provides an in vitro dissolution profile, when measured by the USP Paddle Method at 50 rpm in 900 ml 0.1 N HCl at pH 1.0 and 37 °C, wherein after 0.5 hour 20±20 wt.-%; preferably 20±15 wt.-%; more preferably 20±10 wt.-%; after 4 hours 60±20 wt.-%; preferably 60±15 wt.-%; more preferably 60±10 wt.-%; and after 12 hours at least 60 wt.-%; preferably at least 70 wt.-%; more preferably at least 80 wt.-% of the Tapentadol that was originally contained in the dosage form have been released.
Clause 10: The pharmaceutical dosage form according to any of the preceding clauses, which provides an in vitro dissolution profile, when measured by the USP Paddle Method at 50 rpm in 900 ml 0.1 N HCl at pH 1.0 and 37 °C, wherein- after 1 hour 25±10 wt.-%; - after 2 hours 40±30 wt.-%; - after 4 hours 60±20 wt.-%; - after 8 hours 80±20 wt.-%; of the Tapentadol that was originally contained in the dosage form have been released.
Clause 11: The pharmaceutical dosage form according to any of the preceding clauses, which provides resistance against ethanol induced dose dumping.
Clause 12: The pharmaceutical dosage form according to clause 11, which provides slower in vitro dissolution of Tapentadol in aqueous medium containing ethanol than in aqueous medium not containing ethanol.
Clause 13: The pharmaceutical dosage form according to clause 11 or 12, which provides slower dissolution of Tapentadol in 0.1 N HCl containing 40 vol.-% of ethanol (pH 1.0) than in 0.1 N HCl not containing 40 vol.-% of ethanol (pH 1.0), in each case when measured by the USP Paddle Method at 50 rpm in 900 ml at 37° C.
Clause 14: The pharmaceutical dosage form according to any of the preceding clauses, wherein the weight equivalent dose of Tapentadol that is contained in the pharmaceutical dosage form amounts to 25 mg, 50 mg, or 100 mg, in each case based on the free base of Tapentadol.
Clause 15: The pharmaceutical dosage form according to clause 14, wherein the dosage form has a total weight within the range of from 150 to 750 mg.
Clause 16: The pharmaceutical dosage form according to any of clauses 1 to 13, wherein the weight equivalent dose of Tapentadol that is contained in the pharmaceutical dosage form amounts to 150 mg, 200 mg or 250 mg, in each case based on the free base of Tapentadol.
Clause 17: The pharmaceutical dosage form according to clause 16, wherein the dosage form has a total weight within the range of from 300 to 1200 mg.
Clause 18: The pharmaceutical dosage form according to any of the preceding clauses, wherein the dosage form contains a prolonged release coating comprises a prolonged release coating material selected from the group consisting of hydrophobic cellulose ethers, acrylic polymers, shellac, zein, hydrophobic waxy-type products, and mixtures thereof.
Clause 19: The pharmaceutical dosage form according to clause 18, wherein the prolonged release coating material is selected from the group consisting of ethylcellulose, acrylic acid and methacrylic acid copolymers, methacrylic acid copolymers, methyl methacrylate copolymers, ethoxyethyl methacrylates, cynaoethyl methacrylate, methyl methacrylate, copolymers, methacrylic acid copolymers, methyl methacrylate copolymers, methyl methacrylate copolymers, methyl methacrylate copolymers, methacrylic acid copolymer, aminoalkyl methacrylate copolymer, methacrylic acid copolymers, methyl methacrylate copolymers, poly(acrylic acid), poly(methacrylic acid, methacrylic acid alkylamide copolymer, poly(methyl methacrylate), poly(methacrylic acid) (anhydride), methyl methacrylate, polymethacrylate, methyl methacrylate copolymer, poly(methyl methacrylate), poly(methyl methacrylate) copolymer, polyacrylamide, aminoalkyl methacrylate copolymer, poly(methacrylic acid anhydride), and glycidyl methacrylate copolymers.
Clause 20: The pharmaceutical dosage form according to any of the preceding clauses, wherein Tapentadol is embedded in a prolonged release matrix.
Clause 21: The pharmaceutical dosage form according to clause 20, wherein the prolonged release matrix comprises or essentially consists of at least one prolonged release matrix material selected from the group consisting of hydrophilic or hydrophobic polymers and hydrocarbons.
Clause 22: The pharmaceutical dosage form according to clause 21, wherein the prolonged release matrix comprises or essentially consists of at least one polymer selected from the group consisting of polysaccharides or gums (e.g. xanthan gum, guar gum, karaya gum, locust bean gum, sodium alginate, carob gum, chitosan, polysaccharides of mannose and galactose, pectin, tragacanth, agar-agar); cellulose ethers (e.g. HPMC, HPC, HEC, MC, EC); cellulose esters (e.g. cellulose acetate, cellulose acetate succinate, cellulose acetate phthalate); polyalkylene glycols and polyalkylene oxides; polyvinylalcohol (PVA), cross-linked polyvinylalcohol (PVA), polyvinylpyrrolidone (PVP), cross-linked polyvinylpyrrolidone (PVP), polyvinylpyrrolidone-vinylacetate copolymers, polyvinylchloride (PVC), polyethylene vinyl acetate (PVA), polydimethylsiloxane (PDS), polyether urethane (PEU), polylactic acid (PLA), polyglycolic acid (PGA), polycaprolactone (PCL), polyanhydrides, polyorthoesters; acrylic resins (e.g. cross-linked homopolymers and copolymers of acrylic acid, acrylic acid and methacrylic acid copolymers, methacrylic acid copolymers, methyl methacrylate copolymers, ethoxyethyl methacrylates, cynaoethyl methacrylate, methyl methacrylate, copolymers, methacrylic acid copolymers, methyl methacrylate copolymers, methyl methacrylate copolymers, methyl methacrylate copolymers, methacrylic acid copolymer, aminoalkyl methacrylate copolymer, methacrylic acid copolymers, methyl methacrylate copolymers, poly(acrylic acid), poly(methacrylic acid, methacrylic acid alkylamide copolymer, poly(methyl methacrylate), poly(methacrylic acid) (anhydride), methyl methacrylate, polymethacrylate, methyl methacrylate copolymer, poly(methyl methacrylate), poly(methyl methacrylate) copolymer, polyacrylamide, aminoalkyl methacrylate copolymer, poly(methacrylic acid anhydride), and glycidyl methacrylate copolymers, polyhydroxy ethyl methacrylate (PHEMA), polyacrylamide); and protein derived materials.
Clause 23: The pharmaceutical dosage form according to clause 21 or 22, wherein the prolonged release matrix comprises or essentially consists of at least one hydrocarbon selected from the group consisting of long chain (C8-C50, especially C12-C40) fatty acids, fatty alcohols, glyceryl esters of fatty acids, mineral oils, vegetable oils, and waxes.
Clause 24: The pharmaceutical dosage form according to any of clauses 20 to 23, wherein the prolonged release matrix comprises or essentially consists of a prolonged release matrix material selected from the group consisting of (i) hydroxypropylmethylcellulose (HPMC); (ii) hydroxy-propylcellulose (HPC); (iii) hydroxyethylcellulose (HEC); (iv) microcrystalline cellulose (MCC); (v) ethylcellulose (EC); (vi) polyvinyl acetate (PVAc); (vii) polyvinylpyrrolidone (PVP); (viii) polyvinylpyrrolidone-vinylacetate copolymer (PVP/PVAc); (ix) poly(ethyl acrylate-co-methyl methacrylate-co-trimethylammonioethyl methacrylate chloride); (x) poly(butyl methacrylate-co-(2-dimethylaminoethyl) methacrylate-co-methyl methacrylate); (xi) poly(methyl methacrylate-co-methacrylic acid); (xii) poly(ethyl acrylate-co-methacrylic acid); (xiii) poly(methyl acrylate-co-methyl methacrylate-co-methacrylic acid); (xiv) poly (ethyl acrylate-co-methyl methacrylate); (xv) poly(ethylene oxide) (PEO); (xvi) polyethylene glycol (PEG); (xvii) long chain fatty alcohol having 8 to 50 carbon atoms, preferably 12 to 40 carbon atoms, which may be saturated or unsaturated, linear or branched; (xviii) cetostearyl alcohol; (xix) stearyl alcohol; (xx) cetyl alcohol; (xxi) hydrocarbon selected from the group consisting of long chain fatty acids having 8 to 50 carbon atoms, preferably 12 to 40 carbon atoms, which may be saturated or unsaturated, linear or branched; glyceryl esters of such long chain fatty acids, mineral oils, vegetable oils, and waxes; (xxii) xanthan gum; (xxiii) sodium alginate; (xxiv) guar gum; (xxv) locust bean gum; and any mixtures of the foregoing; wherein preferably, the content of the prolonged release matrix material is within the range of 15±10 wt.-%, or 20±10 wt.-%, or 25±10 wt.-%, or 30±10 wt.-%, or 35±10 wt.-%, or 40±10 wt.-%, or 45±10 wt.-%, or 50±10 wt.-%, or 55±10 wt.-%, or 60±10 wt.-%, or 65±10 wt.-%, or 70±10 wt.-%, or 75±10 wt.-%, or 80±10 wt.-%, in each case relative to the total weight of the dosage form.
Clause 25: The pharmaceutical dosage form according to clause 24, wherein the prolonged release matrix comprises as prolonged release matrix material hydroxypropylmethylcellulose (HPMC) in an amount of from 5.0 to 50 wt.-%, e.g. 15±10 wt.-%, or 20±10 wt.-%, or 25±10 wt.-%, or 30±10 wt.-%, or 35±10 wt.-%, or 40±10 wt.-%, in each case relative to the total weight of the dosage form.
Clause 26: The pharmaceutical dosage form according to clause 24, wherein the prolonged release matrix comprises as prolonged release matrix material hydroxypropylcellulose (HPC) in an amount of from 10 to 50 wt.-%, e.g. 20±10 wt.-%, or 25±10 wt.-%, or 30±10 wt.-%, or 35±10 wt.-%, or 40±10 wt.-%, in each case relative to the total weight of the dosage form.
Clause 27: The pharmaceutical dosage form according to clause 24, wherein the prolonged release matrix comprises as prolonged release matrix material hydroxyethylcellulose (HEC) in an amount of from 5.0 to 50 wt.-%, e.g. 15±10 wt.-%, or 20±10 wt.-%, or 25±10 wt.-%, or 30±10 wt.-%, or 35±10 wt.-%, or 40±10 wt.-%, in each case relative to the total weight of the dosage form.
Clause 28: The pharmaceutical dosage form according to clause 24, wherein the prolonged release matrix comprises as prolonged release matrix material microcrystalline cellulose (MCC) in an amount of from 10 to 70 wt.-%, e.g. 20±10 wt.-%, or 25±10 wt.-%, or 30±10 wt.-%, or 35±10 wt.-%, or 40±10 wt.-%, or 45±10 wt.-%, or 50±10 wt.-%, or 55±10 wt.-%, or 60±10 wt.-%, in each case relative to the total weight of the dosage form.
Clause 29: The pharmaceutical dosage form according to clause 24, wherein the prolonged release matrix comprises as prolonged release matrix material ethylcellulose (EC) in an amount of from 5.0 to 30 wt.-%, e.g. 15±10 wt.-%, or 20±10 wt.-%, in each case relative to the total weight of the dosage form.
Clause 30: The pharmaceutical dosage form according to clause 24, wherein the prolonged release matrix comprises as prolonged release matrix material polyvinyl acetate (PVAc) in an amount of from 25 to 70 wt.-%, e.g. 35±10 wt.-%, or 40±10 wt.-%, or 45±10 wt.-%, or 50±10 wt.-%, or 55±10 wt.-%, or 60±10 wt.-%, in each case relative to the total weight of the dosage form.
Clause 31: The pharmaceutical dosage form according to clause 24, wherein the prolonged release matrix comprises as prolonged release matrix material polyvinylpyrrolidone (PVP) in an amount of from 2.0 to 21 wt.-%, e.g. 10±5.0 wt.-%, or 15±5.0 wt.-%, in each case relative to the total weight of the dosage form.
Clause 32: The pharmaceutical dosage form according to clause 24, wherein the prolonged release matrix comprises as prolonged release matrix material polyvinylpyrrolidone-vinylacetate copolymer (PVP/PVAc) in an amount of from 1.0 to 30 wt.-%, e.g. 15±10 wt.-%, or 20±10 wt.-%, in each case relative to the total weight of the dosage form.
Clause 33: The pharmaceutical dosage form according to clause 24, wherein the prolonged release matrix comprises as prolonged release matrix material poly(ethyl acrylate-co-methyl methacrylate-co-trimethylammonioethyl methacrylate chloride) in an amount of from 5.0 to 45 wt.-%, e.g. 15±10 wt.-%, or 20±10 wt.-%, or 25±10 wt.-%, or 30±10 wt.-%, or 35±10 wt.-%, in each case relative to the total weight of the dosage form.
Clause 34: The pharmaceutical dosage form according to clause 24, wherein the prolonged release matrix comprises as prolonged release matrix material poly(butyl methacrylate-co-(2-dimethyl-aminoethyl) methacrylate-co-methyl methacrylate) in an amount of from 5.0 to 45 wt.-%, e.g. 15±10 wt.-%, or 20±10 wt.-%, or 25±10 wt.-%, or 30±10 wt.-%, or 35±10 wt.-%, in each case relative to the total weight of the dosage form.
Clause 35: The pharmaceutical dosage form according to clause 24, wherein the prolonged release matrix comprises as prolonged release matrix material poly(methyl methacrylate-co-methacrylic acid) in an amount of from 5.0 to 45 wt.-%, e.g. 15±10 wt.-%, or 20±10 wt.-%, or 25±10 wt.-%, or 30±10 wt.-%, or 35±10 wt.-%, in each case relative to the total weight of the dosage form.
Clause 36: The pharmaceutical dosage form according to clause 24, wherein the prolonged release matrix comprises as prolonged release matrix material poly(ethyl acrylate-co-methacrylic acid) in an amount of from 5.0 to 45 wt.-%, e.g. 15±10 wt.-%, or 20±10 wt.-%, or 25±10 wt.-%, or 30±10 wt.-%, or 35±10 wt.-%, in each case relative to the total weight of the dosage form.
Clause 37: The pharmaceutical dosage form according to clause 24, wherein the prolonged release matrix comprises as prolonged release matrix material poly(methyl acrylate-co-methyl methacrylate-co-methacrylic acid) in an amount of from 5.0 to 45 wt.-%, e.g. 15±10 wt.-%, or 20±10 wt.-%, or 25±10 wt.-%, or 30±10 wt.-%, or 35±10 wt.-%, in each case relative to the total weight of the dosage form.
Clause 38: The pharmaceutical dosage form according to clause 24, wherein the prolonged release matrix comprises as prolonged release matrix material poly(ethyl acrylate-co-methyl methacrylate) in an amount of from 5.0 to 45 wt.-%, e.g. 15±10 wt.-%, or 20±10 wt.-%, or 25±10 wt.-%, or 30±10 wt.-%, or 35±10 wt.-%, in each case relative to the total weight of the dosage form.
Clause 39: The pharmaceutical dosage form according to clause 24, wherein the prolonged release matrix comprises as prolonged release matrix material poly(ethylene oxide) (PEO) in an amount of from 25 to 65 wt.-%, e.g. 35±10 wt.-%, or 40±10 wt.-%, or 45±10 wt.-%, or 50±10 wt.-%, or 55±10 wt.-%, in each case relative to the total weight of the dosage form.
Clause 40: The pharmaceutical dosage form according to clause 24, wherein the prolonged release matrix comprises as prolonged release matrix material polyethylene glycol (PEG) in an amount of from 5.0 to 35 wt.-%, e.g. 15±10 wt.-%, or 20±10 wt.-%, or 25±10 wt.-%, in each case relative to the total weight of the dosage form.
Clause 41: The pharmaceutical dosage form according to clause 24, wherein the prolonged release matrix comprises as prolonged release matrix material a long chain fatty alcohol having 8 to 50 carbon atoms, preferably 12 to 40 carbon atoms, which may be saturated or unsaturated, linear or branched, in an amount of from 15 to 40 wt.-%, e.g. 25±10 wt.-%, or 30±10 wt.-%, in each case relative to the total weight of the dosage form.
Clause 42: The pharmaceutical dosage form according to clause 24, wherein the prolonged release matrix comprises as prolonged release matrix material cetostearyl alcohol in an amount of from 15 to 40 wt.-%, e.g. 25±10 wt.-%, or 30±10 wt.-%, in each case relative to the total weight of the dosage form.
Clause 43: The pharmaceutical dosage form according to clause 24, wherein the prolonged release matrix comprises as prolonged release matrix material stearyl alcohol in an amount of from 15 to 40 wt.-%, e.g. 25±10 wt.-%, or 30±10 wt.-%, in each case relative to the total weight of the dosage form. Clause 44: The pharmaceutical dosage form according to clause 24, wherein the prolonged release matrix comprises as prolonged release matrix material cetyl alcohol in an amount of from 15 to 40 wt.-%, e.g. 25±10 wt.-%, or 30±10 wt.-%, in each case relative to the total weight of the dosage form. Clause 45: The pharmaceutical dosage form according to clause 24, wherein the prolonged release matrix comprises as prolonged release matrix material a hydrocarbon selected from the group consisting of long chain fatty acids having 8 to 50 carbon atoms, preferably 12 to 40 carbon atoms, which may be saturated or unsaturated, linear or branched; glyceryl esters of such long chain fatty acids, mineral oils, vegetable oils, and waxes; in each case in an amount of from 5.0 to 70 wt.-%, e.g. 15±10 wt.-%, or 20±10 wt.-%, or 25±10 wt.-%, or 30±10 wt.-%, or 35±10 wt.-%, or 40±10 wt.-%, or 45±10 wt.-%, or 50±10 wt.-%, or 55±10 wt.-%, or 60±10 wt.-%, in each case relative to the total weight of the dosage form.
Clause 46: The pharmaceutical dosage form according to clause 24, wherein the prolonged release matrix comprises as prolonged release matrix material xanthan gum in an amount of from 15 to 40 wt.-%, e.g. 25±10 wt.-%, or 30±10 wt.-%, in each case relative to the total weight of the dosage form. Clause 47: The pharmaceutical dosage form according to clause 24, wherein the prolonged release matrix comprises as prolonged release matrix material sodium alginate in an amount of from 15 to 40 wt.-%, e.g. 25±10 wt.-%, or 30±10 wt.-%, in each case relative to the total weight of the dosage form. Clause 48: The pharmaceutical dosage form according to clause 24, wherein the prolonged release matrix comprises as prolonged release matrix material guar gum in an amount of from 5.0 to 35 wt.-%, e.g. 15±10 wt.-%, or 20±10 wt.-%, or 25±10 wt.-%, in each case relative to the total weight of the dosage form.
Clause 49: The pharmaceutical dosage form according to clause 24, wherein the prolonged release matrix comprises as prolonged release matrix material locust bean gum in an amount of from 5.0 to 35 wt.-%, e.g. 15±10 wt.-%, or 20±10 wt.-%, or 25±10 wt.-%, in each case relative to the total weight of the dosage form.
Clause 50: The pharmaceutical dosage form according to any of the preceding clauses, which is a capsule.
Clause 51: The pharmaceutical dosage form according to any of clauses 1 to 49, which is a tablet. Clause 52: The pharmaceutical dosage form according to clause 51, wherein the tablet is monolithic. Clause 53: The pharmaceutical dosage form according to clause 51 or 52, wherein the tablet has a breaking strength of at least 100 N.
Clause 54: The dosage form according to any of the preceding clauses for use in the treatment of pain, wherein the dosage form is orally administered twice daily.
Clause 55: The dosage form for use according to clause 54, wherein the pain is chronic pain.
Clause 56: The dosage form for use according to clause 54 or 55, which in a patient population of at least 10 patients provides an average value of Tmax within the range of 5.0±3.0 hours after oral administration.
Clause 57: The dosage form for use according to any of clauses 54 to 56, wherein the weight equivalent dose of Tapentadol that is contained in the pharmaceutical dosage form amounts to 50 mg based on the free base of Tapentadol and wherein the dosage form after oral administration in a patient population of at least 10 patients provides an average value of Cmax within the range of 12±3 ng/ml; and/or AUClast within the range of 204±50 ng·h/ml; and/or AUC_{∞} within the range of 214±50 ng·h/ml.
Clause 58: The dosage form for use according to any of clauses 54 to 56, wherein the weight equivalent dose of Tapentadol that is contained in the pharmaceutical dosage form amounts to 100 mg based on the free base of Tapentadol and wherein the dosage form after oral administration in a patient population of at least 10 patients provides an average value of Cmax within the range of 29±6 ng/ml; and/or AUClast within the range of 440±100 ng·h/ml; and/or AUC_{∞} within the range of 447±100 ng·h/ml.
Clause 59: The dosage form for use according to any of clauses 54 to 56, wherein the weight equivalent dose of Tapentadol that is contained in the pharmaceutical dosage form amounts to 150 mg based on the free base of Tapentadol and wherein the dosage form after oral administration in a patient population of at least 10 patients provides an average value of Cmax within the range of 47±9 ng/ml; and/or AUClast within the range of 662±150 ng·h/ml; and/or AUC_{∞} within the range of 665±150 ng·h/ml.
Clause 60: The dosage form for use according to any of clauses 54 to 56, wherein the weight equivalent dose of Tapentadol that is contained in the pharmaceutical dosage form amounts to 200 mg based on the free base of Tapentadol and wherein the dosage form after oral administration in a patient population of at least 10 patients provides an average value of Cmax within the range of 64±12 ng/ml; and/or AUClast within the range of 890±200 ng·h/ml; and/or AUC_{∞} within the range of 895±200 ng·h/ml.
Clause 61: The dosage form for use according to any of clauses 54 to 56, wherein the weight equivalent dose of Tapentadol that is contained in the pharmaceutical dosage form amounts to 250 mg based on the free base of Tapentadol and wherein the dosage form after oral administration in a patient population of at least 10 patients provides an average value of Cmax within the range of 85±15 ng/ml; and/or AUClast within the range of 1141±250 ng·h/ml; and/or AUC_{∞} within the range of 1145±250 ng·h/ml.
Clause 62: A method for the preparation of a pharmaceutical dosage form according to any of the preceding clauses, which contains particles comprising a prolonged release coating, the method comprising the steps of (A) providing inert starter pellets, which contain one or more excipients but no Tapentadol; (B) providing a solution or dispersion of Tapentadol in water or an organic solvent or a mixture thereof, optionally together with the one or more excipients; (C) coating the inert starter pellets provided in step (A) with the solution or dispersion of Tapentadol provided in step (B) thereby obtaining intermediate particles containing an inert core not containing any Tapentadol and a drug coating layer encapsulating said core and comprising essentially the total amount of Tapentadol to be contained in the dosage form, optionally together with the one or more excipients; (D) optionally, drying the intermediate particles obtained in step (C) thereby obtaining dried intermediate particles; (E) providing a solution or dispersion of a prolonged release coating material in water or an organic solvent or a mixture thereof, optionally together with the one or more excipients; (F) coating the intermediate particles obtained in step (C) or the dried intermediate particles obtained in step (D) with the solution or dispersion of prolonged release coating material provided in step (E) thereby obtaining prolonged release particles containing an inert core not containing any Tapentadol, a drug coating layer encapsulating said core and comprising essentially the total amount of Tapentadol, optionally together with the one or more excipients, and a prolonged release coating encapsulating said core and said drug coating layer; (G) optionally, drying the prolonged release particles obtained in step (F) thereby obtained dried prolonged release particles; and (H) either filling the prolonged release particles obtained in step (F) or the dried prolonged release particles obtained in step (G) into capsules; or mixing the prolonged release particles obtained in step (F) or the dried prolonged release particles obtained in step (G) with extra-particle excipients and compressing the mixture into tablets.
Clause 63: A pharmaceutical dosage form that is obtainable by the method according to clause 62.
Clause 64: A method for the preparation of a pharmaceutical dosage form according to any of clauses 1 to 61, which contains particles comprising a prolonged release coating, the method comprising the steps of (A) providing a mixture containing essentially the total amount of Tapentadol to be contained in the dosage form, optionally together with one or more excipients; (B) preparing drug pellets from the mixture provided in step (A) by dry granulation, wet granulation or extrusion; (C) optionally, drying and/or spheronizing the drug pellets prepared in step (B) thereby obtaining dried and/or spheronized drug pellets; (D) providing a solution or dispersion of a prolonged release coating material in water or an organic solvent or a mixture thereof, optionally together with the one or more excipients; (E) coating the drug pellets prepared in step (B) or the dried and/or spheronized drug pellets obtained in step (C) with the solution or dispersion of prolonged release coating material provided in step (D) thereby obtaining prolonged release particles containing a core comprising essentially the total amount of Tapentadol, optionally together with one or more excipients, and a prolonged release coating encapsulating said core; (F) optionally, drying the prolonged release particles obtained in step (E) thereby obtained dried prolonged release particles; and (G) either filling the prolonged release particles obtained in step (E) or the dried prolonged release particles obtained in step (F) into capsules; or mixing the prolonged release particles obtained in step (E) or the dried prolonged release particles obtained in step (F) with extra-particle excipients and compressing the mixture into tablets.
Clause 65: A pharmaceutical dosage form that is obtainable by the method according to clause 64.
Clause 66: A method for the preparation of a pharmaceutical dosage form according to any of clauses 1 to 61, which contains a prolonged release matrix in which the Tapentadol is embedded, the method comprising the steps of (a) providing a mixture containing essentially the total amount of Tapentadol to be contained in the dosage form and at least one prolonged release matrix material, optionally together with one or more excipients; (b) optionally granulating the mixture provided in step (a) thereby obtaining a granulate; (c) optionally mixing the granulate obtained in step (b) with one or more excipients thereby obtaining a granulate mixture; (d) compressing the mixture provided in step (a) or the granulate obtained in step (b) or the granulate mixture obtained in step (c) into tablets; (e) optionally, film coating the tablets compressed in step (d).
Clause 67: The method according to clause 66, wherein compressing in step (d) is performed at a compression force of not more than 20 kN, more preferably not more than 15 kN, still more preferably not more than 10 kN, yet more preferably not more than 9.5 kN, even more preferably not more than 9.0 kN, most preferably not more than 8.75 kN, and in particular not more than 8.5 kN.

### EXAMPLES

The following examples further illustrate the invention but are not to be construed as limiting its scope.

Commercial Tapentadol tablets Palexia® retard contain Tapentadol as hydrochloride salt, whereas the tablet core additionally contains hypromellose, microcrystalline cellulose, highly disperse silicon dioxide, and magnesium stearate. Thus, Palexia® retard tablets contain a prolonged release matrix of hypromellose. These tablets are in accordance with WO 03/035053 A1 and with the comparative examples described hereinafter.

### Example 1 - preparation of Tapentadol dihydrogenphosphate salt:

Three different batches of Tapentadol dihydrogenphosphate were prepared, a batch of essentially pure Tapentadol dihydrogenphosphate hemihydrate, a batch of essentially pure Tapentadol dihydrogenphosphate anhydrate, and a batch comprising a mixture of the two polymorphs (= mixed form).

### Example 1.1 - Tapentadol dihydrogenphosphate mixed form:

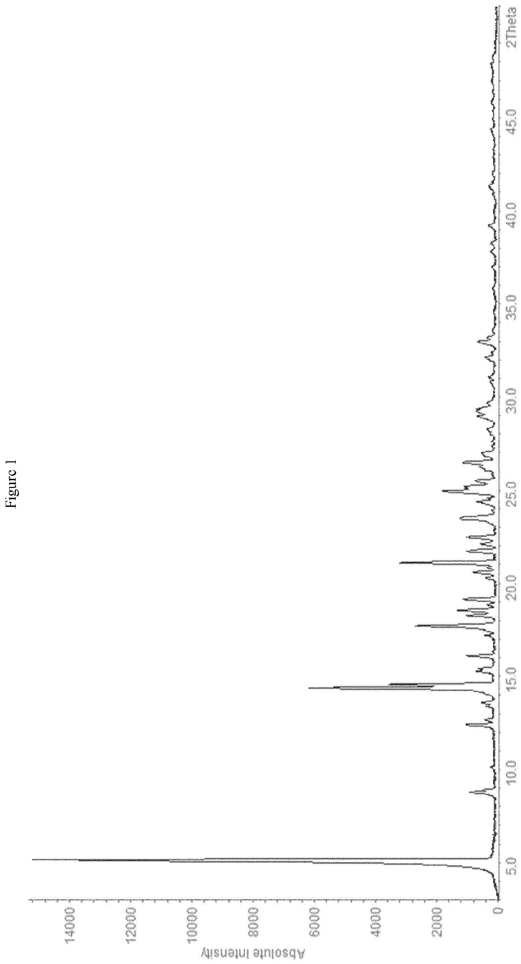

Tapentadol base (200 mg) was thoroughly mixed with phosphoric acid (104 mg, 61.7 µl, 85 wt.-%) using a spatula. The resulting mixture was aged for 4 days at 20 °C to 25 °C to give 292 mg of a white crystalline solid. The obtained product was analyzed by XRPD (Figure 1). A weight loss of 2.8% up to 172 °C was detected by TGA.

### Example 1.2 - Tapentadol dihydrogenphosphate mixed form:

Tapentadol base (7.5 g) was suspended at 20 °C to 25 °C in a mixture of ethyl acetate (81 g, 90 ml) and water (10 mg, 10 µl). The mixture was heated to 55 °C to give a clear solution. After that crystalline Tapentadol dihydrogenphosphate (10 mg) and phosphoric acid (3.9 g, 2.3 ml, 85 wt.-%) were added. Immediately a white suspension is formed. After 45 min of mixing at 60 °C the suspension was cooled to 5 °C in 45min. After further mixing at 5 °C for 45 min the resulting solid was isolated by vacuum filtration and dried (20 °C to 25 °C, vacuum, 2 h). The obtained product (9.5 g, white crystalline powder) was analyzed by XRPD. A weight loss of 3.1% up to 113 °C was detected by TGA.

### Example 1.3 Tapentadol dihydrogenphosphate mixed form:

Tapentadol base (7.5 g) was dissolved at 20 °C to 25 °C in a mixture of tetrahydrofuran (80 g, 90 ml) and water (10 mg, 10 µl). The mixture was heated to 55 °C to give a clear solution. After that crystalline Tapentadol dihydrogenphosphate (10 mg) and phosphoric acid (3.9 g, 2.3 ml, 85 wt.-%) were added. Immediately a white suspension is formed. After 45 min of mixing at 60 °C the suspension was cooled to 5 °C in 45 min. After further mixing at 5 °C for 45 min the resulting solid was isolated by vacuum filtration and dried (20 °C to 25 °C, vacuum, 2 h). The obtained product (9.6 g, white crystalline powder) was analyzed by XRPD. A weight loss of 1.9% up to 120 °C was detected by TGA.

### Example 1.4 - Tapentadol dihydrogenphosphate hemihydrate:

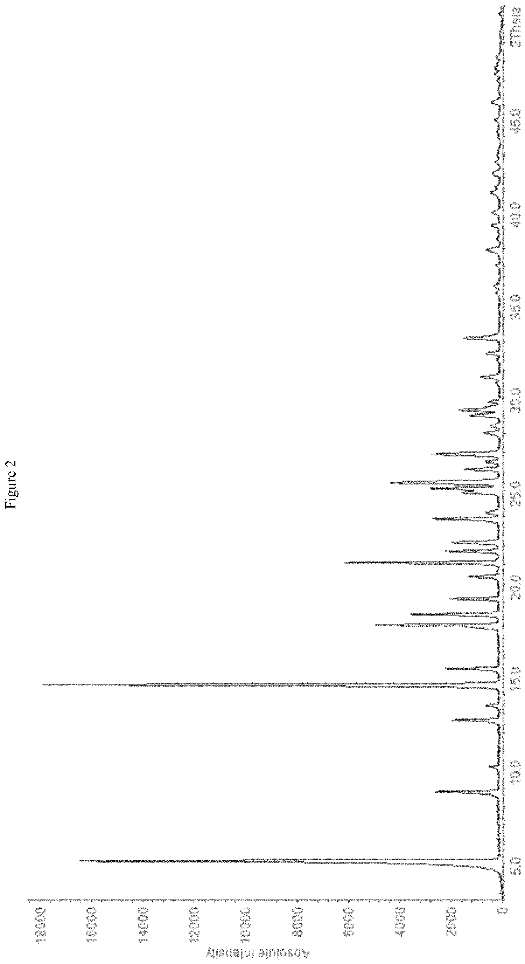

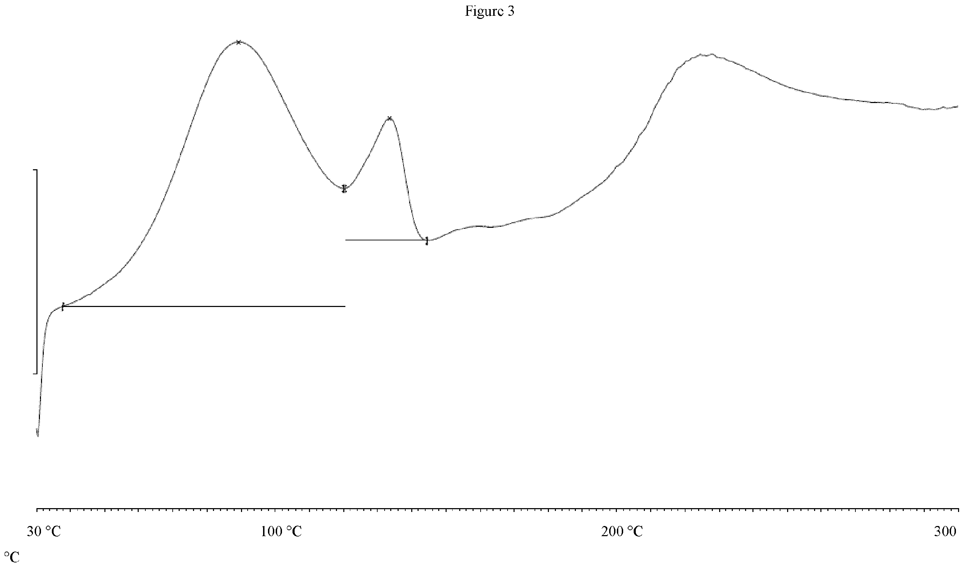

Tapentadol base (497 mg) was dissolved at 20 °C to 25 °C in a mixture of acetone (2.72 g, 3.44 ml) and water (50 mg, 50 µl) to give a clear solution. Phosphoric acid (259 mg, 153 µl, 85 wt.-%) was added and after a short time the clear solution became a suspension. The suspension was mixed for 30 min at 20 °C to 25 °C, after that cooled to 5 °C and further mixed at 5 °C for 30 min. The resulting solid was isolated by vacuum filtration and dried (20 °C to 25 °C, vacuum, 30 min). The obtained product (404 g, white crystalline powder) was analyzed by XRPD (Figure 2) and DSC (Figure 3). The first DSC peak had a normalized integral of 149.2 J·g⁻¹, with an onset temperature of 56.7 °C and a peak temperature of 89.2 °C. The second DSC peak had a normalized integral of 22.0 J·g⁻¹, with an onset temperature of 130.1 °C and a peak temperature of 133.4 °C. At about 200 °C, decomposition occurred. A weight loss of 3.7% up to 119 °C was detected by TGA.

### Example 1.5 - Tapentadol dihydrogenphosphate hemihydrate (coarse material):

Tapentadol base (60 g) was dissolved at 20 °C to 25 °C in a mixture of tetrahydrofuran (1.5 kg, 1.64 1) and water (82 g, 82 ml). Phosphoric acid (31.25 g, 18.5 ml, 85 wt.-%) and crystalline Tapentadol dihydrogenphosphate (5 mg) were added. During the addition the solution became a white suspension. The resulting suspension was cooled to 10 °C in 30 min. The mixture was heated to 35 °C and mixed for 30 min followed by cooling to 5 °C in 30 min. The suspension was mixed at 5 °C for 45 min. The resulting solid was isolated by vacuum filtration and dried (20 °C to 25 °C, vacuum, 2 h). The obtained product (84.6 g, white crystalline solid) was analyzed by XRPD. A weight loss of 4.5% up to 113 °C was detected by TGA.

### Example 1.6 - Tapentadol dihydrogenphosphate anhydrate:

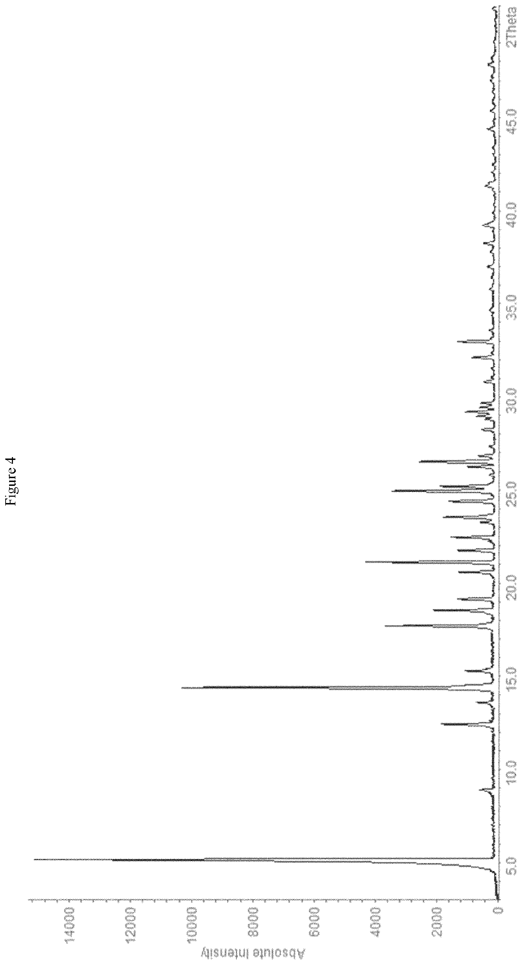

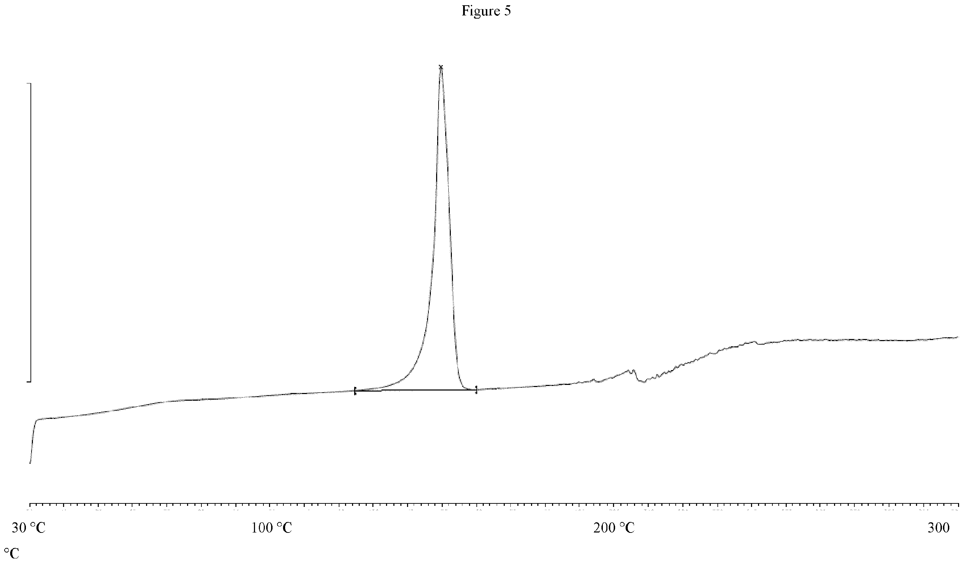

Tapentadol base (150 mg) was suspended at 20 °C to 25 °C in 1-butanol (1.2 g, 1.5 ml). The suspension was heated to 50 °C to give a clear solution. Phosphoric acid (78.1 mg, 46.3 µl, 85 wt.-%) and crystalline Tapentadol dihydrogenphosphate (5 mg) were added at 50 °C. During the addition the clear solution became a white suspension. The resulting suspension was heated to 115 °C. To keep the suspension mixable 1-butanol (400 mg, 0.5 ml) was added and mixing at 115 °C was continued for 45 min. The mixture was then cooled to 30 °C in 3 h. The resulting solid was isolated by vacuum filtration and dried (20 °C to 25 °C, vacuum, 30 min). The obtained product (157 mg, white crystalline powder) was analyzed by XRPD (Figure 4) and DSC (Figure 5). The DSC peak had a normalized integral of 72.1 J·g⁻¹, with an onset temperature of 146.5 °C and a peak temperature of 149.1 °C. At about 200 °C, decomposition occurred. A weight loss of 0.9% up to 134 °C was detected by TGA.

### Example 1.7 - Tapentadol dihydrogenphosphate anhydrate:

Tapentadol dihydrogenphosphate (500 mg, mix of hemihydrate and anhydrate) was suspended at 20 °C to 25 °C in 2-butanone (4.8 g, 6 ml) and water (5 mg, 5 µl). The reaction mixture was heated to 60 °C and mixed for 3 h. The resulting solid was isolated by hot filtration using vacuum and dried (20 °C to 25 °C, vacuum, 30 min). The obtained product (452 mg, white crystalline powder) was analyzed by XRPD. A weight loss of 1.0% up to 129 °C was detected by TGA.

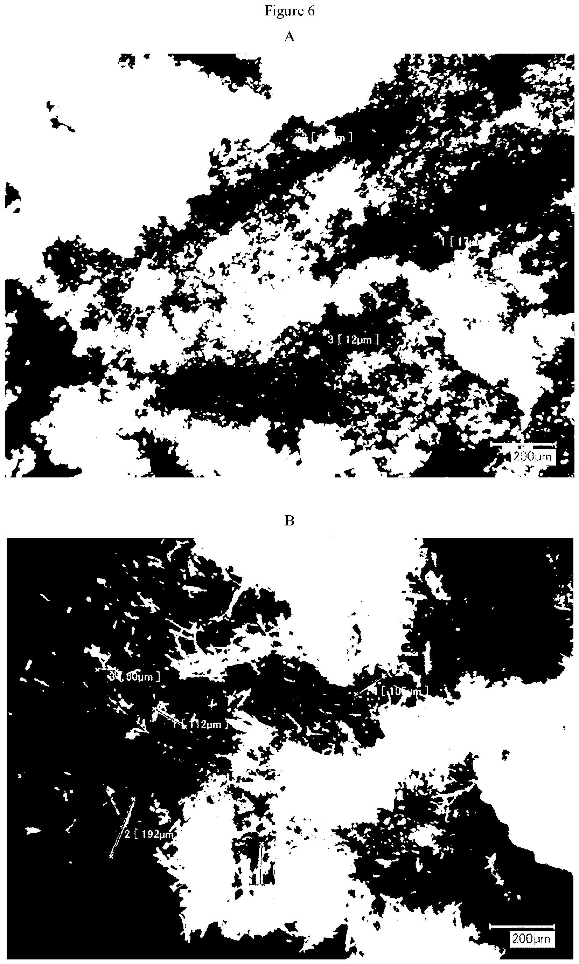

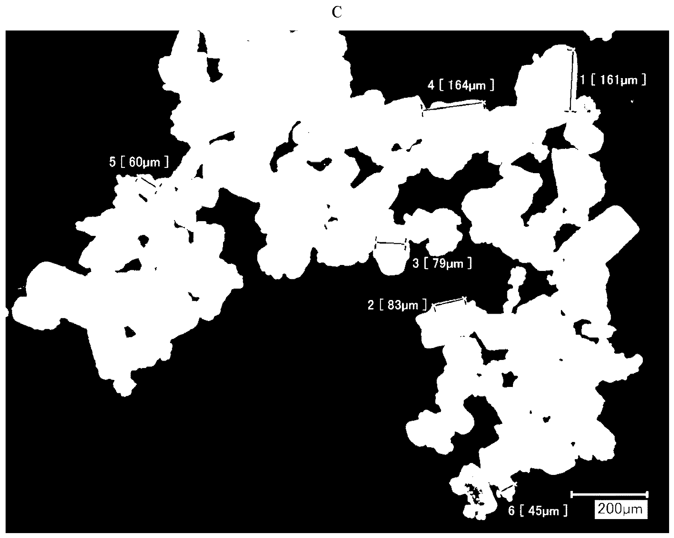

Figure 6 shows three photographs of different salts of Tapentadol that were used for manufacturing tablets. Figure 6A shows relatively fine particles of the mixed form of Tapentadol dihydrogenphosphate (average particle size upon visual inspection about 10-20 µm). Figure 6B shows relatively coarse particles of the pure hemihydrate of Tapentadol dihydrogenphosphate according to Example 1.5 (needle like crystals, average particle size upon visual inspection about 100-200 µm). Figure 6C shows relatively coarse particles of Tapentadol hydrochloride (average particle size upon visual inspection about 100-150 µm).

### Example 2 - intrinsic dissolution (dissolution rate):

100 mg of Tapentadol, either in form of the hydrochloride salt or the dihydrogenphosphate salt (mixed form [batch with fine particles], pure hemihydrate or pure anhydrate) were compressed at a compressing force of 200 kg (gravitational) for 1 minute at a surface of 0.5 cm². The thus obtained compressed samples were investigated for their dissolution rate in a Wood's apparatus at 37 °C in 900 mL of dissolution medium at different pH values and a rotational speed of the paddle of 50 rpm.

The experimental results for Tapentadol hydrochloride and for Tapentadol dihydrogenphosphate (mixed form) are compiled in the table here below:

| [%] | Tapentadol hydrochloride (comparative) | | | | | Tapentadol dihydrogenphosphate (mixed form) (inventive) | | | |
|---|---|---|---|---|---|---|---|---|---|
| min. | pH 1.0 | pH 4.5 | pH 7.4 | pH 6.8 | | pH 1.0 | pH 4.5 | pH 7.4 | pH 6.8 |
| 0 | 0 | 0 | 0 | 0 | | 0 | 0 | 0 | 0 |
| 1 | 52 | 60 | 27 | 38 | | 14 | 12 | 12 | 11 |
| 3 | 95 | 91 | 65 | 79 | | 35 | 30 | 30 | 29 |
| 5 | 99 | 95 | 80 | 92 | | 56 | 49 | 46 | 48 |
| 7 | 99 | 95 | 89 | 94 | | 78 | 73 | 63 | 66 |
| 9 | 99 | 96 | 92 | 94 | | 84 | 86 | 78 | 77 |

It becomes clear from the comparative experimental data in the above table that Tapentadol dihydrogenphosphate (mixed form) under all experimental conditions, i.e. at all tested pH values, has a significantly slower dissolution rate as Tapentadol hydrochloride.

The experimental results for Tapentadol dihydrogenphosphate hemihydrate and for Tapentadol dihydrogenphosphate anhydrate are compiled in the table here below

| [%] | Tapentadol dihydrogenphosphate (inventive) | | | |
|---|---|---|---|---|
| | hemihydrate | | anhydrate | |
| min | pH 6.8 | pH 1.2 | pH 6.8 | pH 1.2 |
| 0 | 0 | 0 | 0 | 0 |
| 1 | 12 | 14 | 13 | 15 |
| 3 | 36 | 37 | 36 | 37 |
| 5 | 66 | 54 | 66 | 57 |
| 7 | 80 | 72 | 90 | 75 |
| 9 | 83 | 84 | 96 | 85 |

It becomes clear from the comparative experimental data in the above table that the slower dissolution rate of Tapentadol dihydrogenphosphate compared to Tapentadol hydrochloride is independent from the polymorphic form of Tapentadol dihydrogenphosphate.

### Example 3 - thermodynamic solubility

The thermodynamic solubility of Tapentadol hydrochloride and of Tapentadol dihydrogenphosphate (mixed form, batch with fine particles) was determined as saturation solubility in various media at various pH values. The solutions were stirred for 24 hours at 25 °C and the pH values of the solutions at the start and at the end of the experiments were measured. The dissolved amount of Tapentadol was quantified by HPLC (free base of Tapentadol). The experimental results are compiled in the table here below:

| | Tapentadol hydrochloride (comparative) | | | | Tapentadol dihydrogenphosphate (mixed form) (inventive) | | |
|---|---|---|---|---|---|---|---|
| | pH | | assay expressed as base | | pH | | assay expressed as base |
| | start | end | g/100 mL | | start | end | g/100 mL |
| 0.1 M HCl | 1.11 | 0.83 | 31.2 | | 1.11 | 2.55 | 24.3 |
| acetate pH 4.5 | 4.54 | 4.29 | 32.7 | | 4.54 | 2.81 | 21.2 |
| water | n.d. | 4.63 | 33.0 | | nd | 2.75 | 21.4 |
| citrate pH 6.8 | 6.83 | 6.17 | 31.8 | | 6.83 | 3.15 | 19.3 |
| SIF_sp pH 6.8 | 6.82 | 6.23 | 32.4 | | 6.82 | 2.81 | 20.2 |
| citrate pH 7.4 | 7.39 | 6.33 | 31.9 | | 7.39 | 3.16 | 19.0 |
| 0.15N NaOH | 13.08 | 8.21 | 6.6 | | 13.08 | 8.09 | 6.5 |

| | | | | | | | |
|---|---|---|---|---|---|---|---|
| n.d. = not determined | | | | | | | |

It becomes clear from the comparative experimental data in the above table that under all tested conditions the thermodynamic solubility of Tapentadol dihydrogenphosphate (mixed form) is below that of Tapentadol hydrochloride.

### Example 4 - in vitro dissolution profiles of pharmaceutical dosage forms:

Tablets having the following composition were prepared by mixing all ingredients and compressing the resultant mixtures:

| | comparative | | inventive | | |
|---|---|---|---|---|---|
| | C-1 | C-2 | I-1 | I-2 | I-3 |
| [mg] | | | | | |
| Tapentadol hydrochloride¹ | 250.00 | 25.00 | - | - | - |
| fine Tapentadol dihydrogenphosphate^{1,2,3} | - | - | 250.00 | 25.00 | - |
| coarse Tapentadol dihydrogenphosphate⁴ | - | - | - | - | 250.00 |
| hydroxypropylmethylcellulose 100,000 mPa·s Typ 2208 | 100.00 | 100.00 | 100.00 | 100.00 | 100.00 |
| silicified microcrystalline cellulose (Prosolv HD90) | 304.80 | 79.79 | 304.80 | 79.79 | 304.80 |
| magnesium stearate | 4.00 | 1.09 | 4.00 | 1.09 | 4.00 |
| mean breaking strength, Ph. Eur. 2.9.8. [N] | 222 | 209 | 222 | 218 | 205 |
| mean tableting force upper punch [kN] | 12.8 | 9.3 | 7.4 | 6.3 | 8.3 |
| mean tableting force lower punch [kN] | 11.3 | 8.1 | 5.0 | 4.9 | 4.3 |

| | | | | | |
|---|---|---|---|---|---|
| ¹ equivalent dose based on the free base of Tapentadol ² mixed form ³ relatively fine particle size (average diameter about 10-20 µm upon visual inspection) ⁴ relatively coarse particle size (average diameter about 100-200 µm upon visual inspection) | | | | | |

The *in vitro* dissolution of Tapentadol from the tablets (drug load 250 mg, C-1, 1-1 and 1-3) was measured in a paddle apparatus in accordance with Ph. Eur., equipped with sinker, at a rotational speed of 50 rpm at 37 °C in 900 ml of different dissolution media having different pH values. The experimental results are compiled in the table here below:

| [%] | Tapentadol hydrochloride (n=3): | | | | | Tapentadol dihydrogenphosphate (n=3): | | | | |
|---|---|---|---|---|---|---|---|---|---|---|
| | C-1 | | | | | I-1 | | | | I-3 |
| time [min] | pH 6.8 | pH 4.5 | pH 1.0 | pH 1.0 40 vol.-% ethanol | | pH 6.8 | pH 4.5 | pH 1.0 | pH 1.0 40 vol.-% ethanol | pH 4.5 |
| 0 | 0 | 0 | 0 | 0 | | 0 | 0 | 0 | 0 | 0 |
| 30 | 17 | 19 | 18 | 14 | | 14 | 15 | 16 | 10 | 15 |
| 60 | 26 | 28 | 27 | 22 | | 23 | 23 | 26 | 18 | 23 |
| 90 | 33 | 36 | 34 | 28 | | 30 | 30 | 33 | 24 | 30 |
| 120 | 39 | 42 | 40 | 33 | | 35 | 36 | 39 | 29 | 36 |
| 150 | 45 | 48 | 46 | 38 | | 41 | 41 | 45 | 33 | 41 |
| 180 | 49 | 54 | 51 | 42 | | 45 | 46 | 50 | 36 | 46 |
| 210 | 54 | 58 | 55 | 46 | | 49 | 50 | 54 | 40 | 50 |
| 240 | 58 | 63 | 60 | 50 | | 53 | 54 | 58 | 43 | 54 |
| 270 | 62 | 67 | 64 | 53 | | 57 | 58 | 62 | 46 | 58 |
| 300 | 66 | 71 | 67 | 57 | | 61 | 62 | 66 | 48 | 61 |
| 330 | 69 | 74 | 71 | 60 | | 64 | 65 | 69 | 51 | 64 |
| 360 | 72 | 77 | 74 | 63 | | 67 | 68 | 72 | 53 | 67 |
| 390 | 75 | 80 | 77 | 65 | | 70 | 71 | 75 | 55 | 70 |
| 420 | 78 | 83 | 79 | 68 | | 73 | 74 | 78 | 58 | 73 |
| 450 | 80 | 85 | 82 | 70 | | 75 | 77 | 80 | 60 | 76 |
| 480 | 82 | 87 | 84 | 73 | | 78 | 79 | 83 | 62 | 78 |
| 510 | 84 | 89 | 86 | 75 | | 80 | 82 | 85 | 64 | 81 |
| 540 | 86 | 91 | 88 | 77 | | 82 | 84 | 87 | 65 | 83 |
| 570 | 88 | 92 | 89 | 79 | | 84 | 86 | 88 | 67 | 86 |
| 600 | 89 | 93 | 91 | 81 | | 86 | 88 | 90 | 69 | 88 |
| 630 | 90 | 94 | 92 | 82 | | 88 | 90 | 91 | 71 | 90 |
| 660 | 92 | 95 | 93 | 84 | | 89 | 91 | 93 | 72 | 91 |
| 690 | 93 | 96 | 94 | 85 | | 91 | 92 | 94 | 74 | 93 |
| 720 | 93 | 96 | 95 | 87 | | 92 | 93 | 95 | 76 | 94 |

It becomes clear from the above data that the *in vitro* dissolution profile of the inventive tablets according to Example 1-1 and 1-3 at pH 4.5 are nearly identical. Thus, as demonstrated, the particle size of the salt of Tapentadol with phosphoric acid (Example 1-1 relatively fine, Example 1-3 relatively coarse), does not significantly alter the *in vitro* dissolution profile.

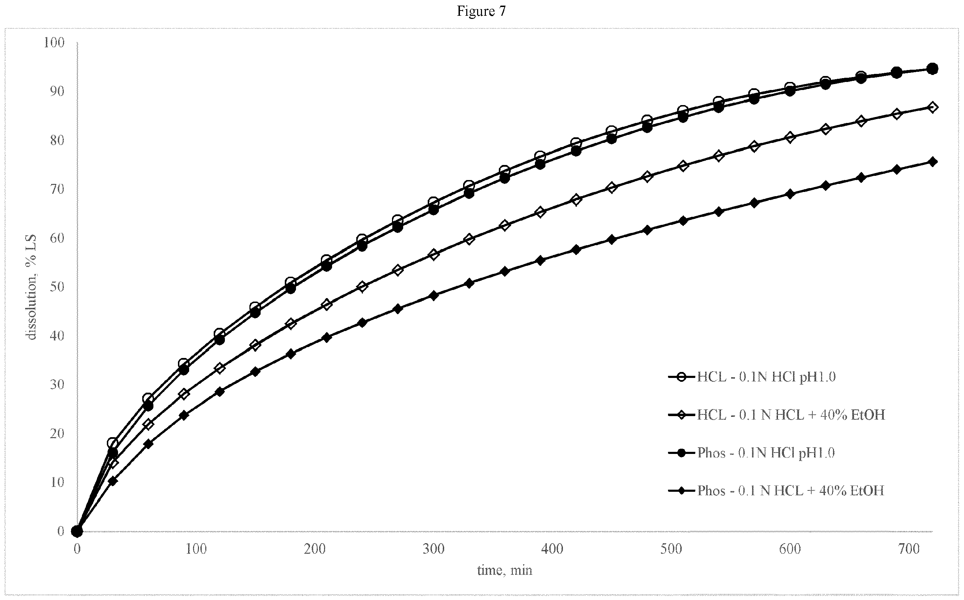

The *in vitro* dissolution profiles in 0.1 N HCl (pH 1.0) and in 0.1 N HCl containing 40 vol.-% aqueous ethanol (pH 1.0) are shown in Figure 7. It becomes clear from Figure 7 that at in 0.1 N HCl (pH 1.0), the *in vitro* dissolution of Tapentadol dihydrogenphosphate is very similar to the *in vitro* dissolution of Tapentadol hydrochloride. Further, it becomes clear from Figure 7 that in 0.1 N HCl (pH 1.0) containing 40 vol.-% ethanol, *in vitro* dissolution is retarded compared to pH 1.0 for both, Tapentadol dihydrogenphosphate and Tapentadol hydrochloride. Thus, the dosage forms show no ethanol induced dose dumping and even provide additional retardation in aqueous ethanol.

However, this additional retardant effect is significantly stronger for Tapentadol dihydrogenphosphate as compared to Tapentadol hydrochloride. As the dosage forms contained the same excipients in the same amounts, this effect is attributable to the salt form of Tapentadol (dihydrogenphosphate vs. hydrochloride). Thus, as demonstrated, Tapentadol dihydrogenphosphate provides additional safety features with regard to concomitant use of ethanol, e.g. alcoholic beverages.

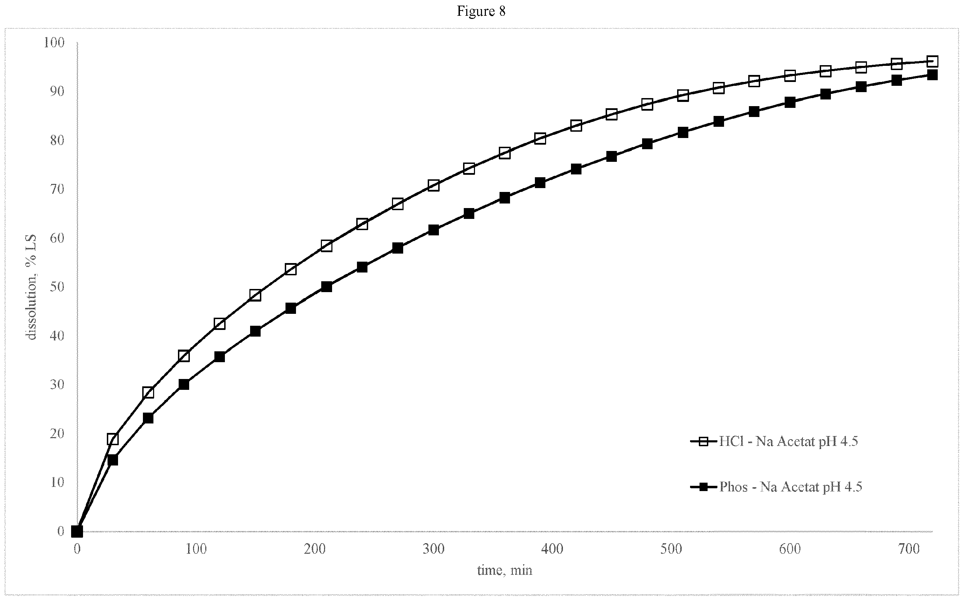

The *in vitro* dissolution profiles at pH 4.5 in aqueous buffer (without ethanol) are shown in Figure 8. It becomes clear from Figure 8 that at pH 4.5 the difference of the *in vitro* dissolution of Tapentadol dihydrogenphosphate compared to Tapentadol hydrochloride is more pronounced than at pH 6.8 (see Figure 7, Δ vs. ▲). At pH 4.5, a release of e.g. 70% for Tapentadol hydrochloride is achieved significantly earlier than for Tapentadol dihydrogenphosphate the time difference being more than one hour. As the dosage forms contained the same excipients in the same amounts, it can be concluded that for Tapentadol dihydrogenphosphate less prolonged release matrix material (e.g. HPMC) will be needed in order to achieve the same *in vitro* dissolution as Tapentadol hydrochloride. Thus, as demonstrated, the slower intrinsic dissolution (dissolution rate) of Tapentadol dihydrogenphosphate allows for reducing the amount of excipients, especially the quantity of prolonged release matrix material, in order to achieve the desired *in vitro* dissolution profile.

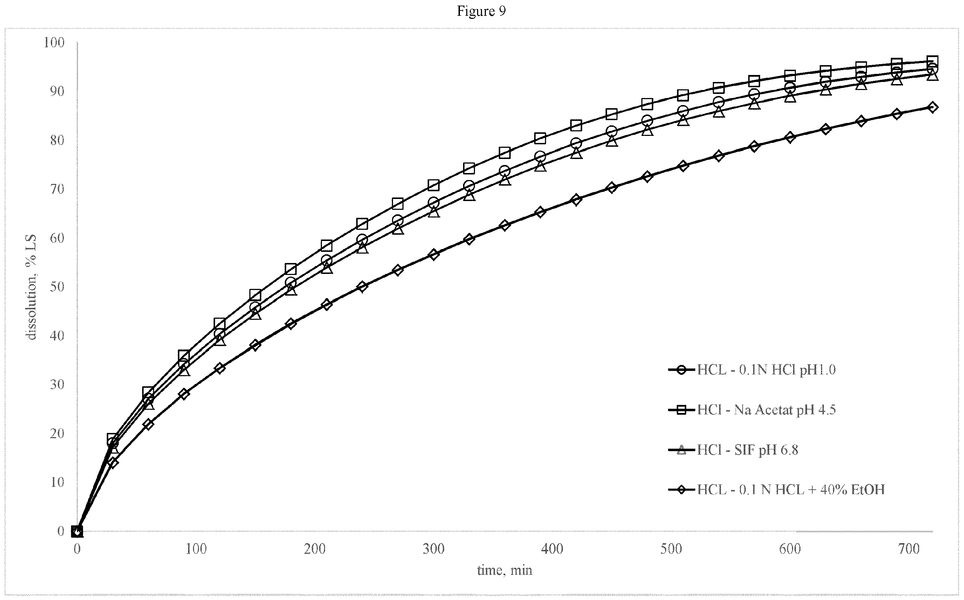

Figure 9 compares the *in vitro* dissolution profiles of a conventional tablet containing Tapentadol hydrochloride at pH 1.0, pH 4.5, pH 6.8 in each case in aqueous buffer without ethanol and in 0.1 N HCl with 40 vol.-% ethanol (pH 1.0).

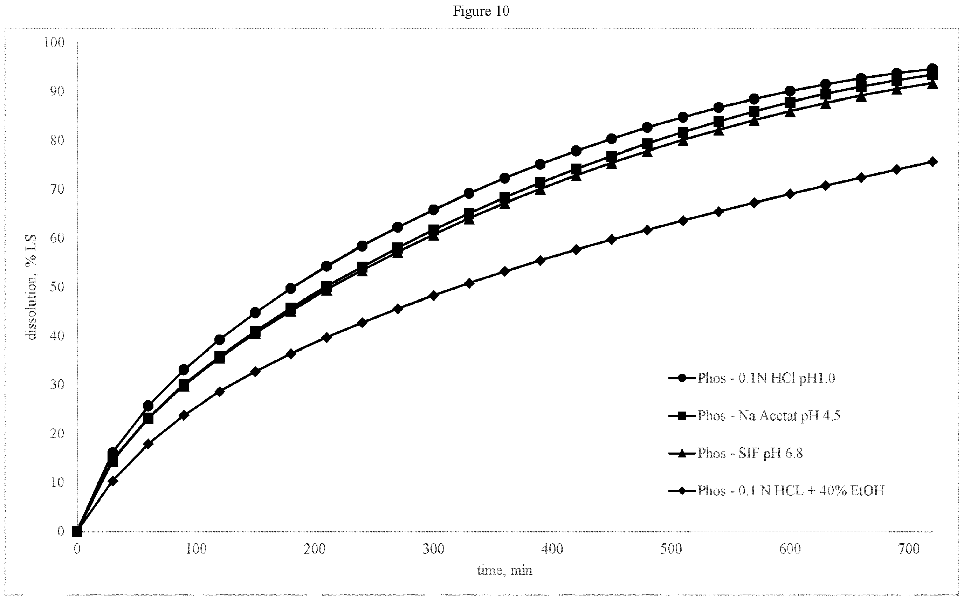

Figure 10 compares the *in vitro* dissolution profiles of the inventive tablet containing salt of Tapentadol with phosphoric acid at pH 1.0, pH 4.5, pH 6.8 in each case in aqueous buffer without ethanol and in 0.1 N HCl with 40 vol.-% ethanol (pH 1.0).

### Example 5 - in vitro dissolution profiles of pharmaceutical dosage forms:

Tablets having the following composition were prepared by mixing all ingredients and compressing the resultant mixtures:

| | comparative | inventive |
|---|---|---|
| [mg] | C-3 | I-4 |
| Tapentadol hydrochloride¹ | 250.00 | - |
| Tapentadol dihydrogenphosphate^{1,2,3} | - | 250.00 |
| Kollidon® SR | 320.00 | 320.00 |
| Aerosil 200 | 6.80 | 6.80 |
| magnesium stearate | 3.20 | 3.20 |
| mean breaking strength, Ph. Eur. 2.9.8. [N] | 237 | 230 |
| mean tableting force upper punch [kN] | 12.4 | 8.1 |
| mean tableting force lower punch [kN] | 9.9 | 4.4 |

| | | |
|---|---|---|
| ¹ equivalent dose based on the tree base or Tapentadol ² mixed form ³ relatively fine particle size (average diameter about 20 µm upon visual inspection) | | |

The *in vitro* dissolution of Tapentadol from the tablets (drug load 250 mg, 1-4) was measured in a paddle apparatus in accordance with Ph. Eur., equipped with sinker, at a rotational speed of 50 rpm at 37 °C in 900 ml of 0.1 N HCl without ethanol (pH 1.0) and in 0.1 N HCl with 40 vol.-% ethanol (pH 1.0). The experimental results are compiled in the table here below:

| time [min] | pH 1.0 [%] | pH 1.0 40 vol.-% ethanol [%] |
|---|---|---|
| 0 | 0 | 0 |
| 30 | 30 | 14 |
| 60 | 40 | 21 |
| 90 | 47 | 26 |
| 120 | 53 | 30 |
| 150 | 58 | 34 |
| 180 | 63 | 38 |
| 210 | 67 | 42 |
| 240 | 71 | 45 |
| 270 | 75 | 49 |
| 300 | 78 | 53 |
| 330 | 81 | 56 |
| 360 | 84 | 60 |
| 390 | 87 | 63 |
| 420 | 89 | 67 |
| 450 | 91 | 71 |
| 480 | 92 | 76 |
| 510 | 94 | 81 |
| 540 | 95 | 85 |
| 570 | 96 | 87 |
| 600 | 97 | 89 |
| 630 | 97 | 91 |
| 660 | 98 | 93 |
| 690 | 98 | 94 |
| 720 | 98 | 95 |

It becomes clear from the above data that in 0.1 N HCl (pH 1.0) containing 40 vol.-% ethanol, *in vitro* dissolution is retarded compared to pH 1.0. Thus, the dosage form shows no ethanol induced dose dumping and even provides additional retardation in aqueous ethanol. Thus, this additional retardant effect is attributable to the salt of Tapentadol with phosphoric acid and is observed in different dosage forms, e.g. in prolonged release matrices based upon hypromellose (see examples 1-1 and 1-3) and also in prolonged release matrices based upon Kollidon® SR (blend containing polyvinyl acetate and povidone).

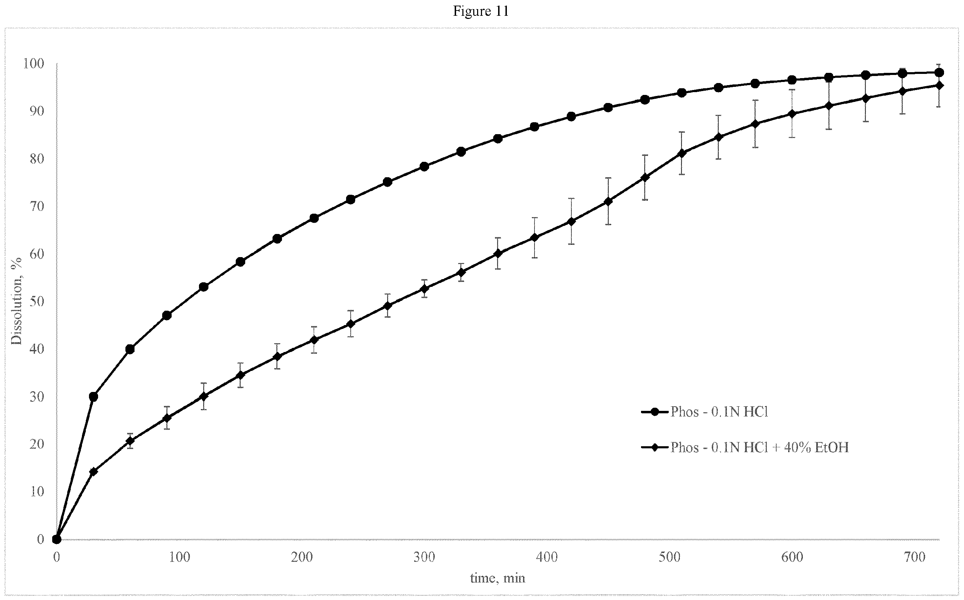

Figure 11 compares the *in vitro* dissolution profiles of an inventive tablet containing salt of Tapentadol with phosphoric acid in 0.1 N HCl (pH 1.0) without ethanol and in 0.1 N HCl (pH 1.0) in 40 vol.-% ethanol.

## Claims

1. A pharmaceutical dosage form comprising Tapentadol for oral administration twice daily;
wherein Tapentadol is present as a salt with phosphoric acid;
wherein the weight equivalent dose of Tapentadol that is contained in the pharmaceutical dosage form is within the range of from 10 to 300 mg based on the free base of Tapentadol;
wherein Tapentadol is embedded in a prolonged release matrix; and
wherein the dosage form provides an *in vitro* dissolution profile, when measured by the USP Paddle Method at 50 rpm in 900 ml aqueous phosphate buffer at pH 6.8 at 37° C, wherein
- after 0.5 hour 20±20 wt.-%;
- after 4 hours 60±20 wt.-%; and
- after 12 hours at least 60 wt.-%;
of the Tapentadol that was originally contained in the dosage form have been released.

2. The pharmaceutical dosage form according to claim 1, wherein the salt of Tapentadol with phosphoric acid is the dihydrogenphosphate salt of Tapentadol, a solvate, an ansolvate, and/or a polymorph thereof, a crystalline form and/or amorphous form thereof.

3. The pharmaceutical dosage form according to claim 1 or 2, which is a tablet.

4. The pharmaceutical dosage form according to claim 3, wherein the tablet is monolithic.

5. The pharmaceutical dosage form according to claim 3 or 4, wherein the tablet has a breaking strength of at least 100 N, determined according to Ph. Eur. 2.9.8.

6. The pharmaceutical dosage form according to any of the preceding claims, which provides slower dissolution of Tapentadol in 0.1 N HCl containing 40 vol.-% of ethanol (pH 1.0) than in 0.1 N HCl not containing 40 vol.-% of ethanol (pH 1.0), in each case when measured by the USP Paddle Method at 50 rpm in 900 ml at 37° C.

7. The pharmaceutical dosage form according to any of the preceding claims, wherein the dosage form provides an *in vitro* dissolution profile, when measured by the USP Paddle Method at 50 rpm in 900 ml (i) aqueous phosphate buffer at pH 6.8, (ii) aqueous buffer at pH 4.5, and/or (iii) 0.1 N HCl at pH 1.0, in each case at 37° C, wherein
- after 0.5 hour 20±15 wt.-%;
- after 4 hours 60±15 wt.-%; and
- after 12 hours at least 70 wt.-%;
of the Tapentadol that was originally contained in the dosage form have been released.

8. The pharmaceutical dosage form according to any of the preceding claims, wherein the prolonged release matrix comprises or essentially consists of a prolonged release matrix material selected from the group consisting of
(i) hydroxypropylmethylcellulose (HPMC);
(ii) hydroxypropylcellulose (HPC);
(iii) hydroxyethylcellulose (HEC);
(iv) microcrystalline cellulose (MCC);
(v) ethylcellulose (EC);
(vi) polyvinyl acetate (PVAc);
(vii) polyvinylpyrrolidone (PVP);
(viii) polyvinylpyrrolidone-vinylacetate copolymer (PVP/PVAc);
(ix) poly(ethyl acrylate-co-methyl methacrylate-co-trimethylammonioethyl methacrylate chloride);
(x) poly(butyl methacrylate-co-(2-dimethylaminoethyl) methacrylate-co-methyl methacrylate);
(xi) poly(methyl methacrylate-co-methacrylic acid);
(xii) poly(ethyl acrylate-co-methacrylic acid);
(xiii) poly(methyl acrylate-co-methyl methacrylate-co-methacrylic acid);
(xiv) poly(ethyl acrylate-co-methyl methacrylate);
(xv) poly(ethylene oxide) (PEO);
(xvi) polyethylene glycol (PEG);
(xvii) long chain fatty alcohol having 8 to 50 carbon atoms, preferably 12 to 40 carbon atoms, which may be saturated or unsaturated, linear or branched;
(xviii) cetostearyl alcohol;
(xix) stearyl alcohol;
(xx) cetyl alcohol;
(xxi) hydrocarbon selected from the group consisting of long chain fatty acids having 8 to 50 carbon atoms, preferably 12 to 40 carbon atoms, which may be saturated or unsaturated, linear or branched; glyceryl esters of such long chain fatty acids, mineral oils, vegetable oils, and waxes;
(xxii) xanthan gum;
(xxiii) sodium alginate;
(xxiv) guar gum;
(xxv) locust bean gum; and
any mixtures of the foregoing.

9. The pharmaceutical dosage form according to claim 8, wherein the content of the prolonged release matrix material is within the range of 15±10 wt.-%, or 20±10 wt.-%, or 25±10 wt.-%, or 30±10 wt.-%, or 35±10 wt.-%, or 40±10 wt.-%, or 45±10 wt.-%, or 50±10 wt.-%, or 55±10 wt.-%, or 60±10 wt.-%, or 65±10 wt.-%, or 70±10 wt.-%, or 75±10 wt.-%, or 80±10 wt.-%, in each case relative to the total weight of the dosage form.

10. The pharmaceutical dosage form according to any of the preceding claims for use in the treatment of pain.

11. The pharmaceutical dosage form for use according to claim 10, wherein the dosage form is orally administered.

12. The pharmaceutical dosage form for use according to claim 10 or 11, wherein the dosage form is administered twice daily.

13. The pharmaceutical dosage form for use according to any of claims 10 to 12, which after oral administration provides plasma levels of Tapentadol providing pain relief for a duration of at least 6 hours.

14. The pharmaceutical dosage form for use according to any of claims 10 to 13, wherein the pain is chronic pain.

15. A method for the preparation of a pharmaceutical dosage form according to any of claims 1 to 14, the method comprising the steps of
(a) providing a mixture containing essentially the total amount of Tapentadol to be contained in the dosage form and at least one prolonged release matrix material, optionally together with one or more excipients;
(b) optionally granulating the mixture provided in step (a) thereby obtaining a granulate;
(c) optionally mixing the granulate obtained in step (b) with one or more excipients thereby obtaining a granulate mixture;
(d) compressing the mixture provided in step (a) or the granulate obtained in step (b) or the granulate mixture obtained in step (c) into tablets;
(e) optionally, film coating the tablets compressed in step (d).
